# EUROPEAN PATENT APPLICATION

(11) **EP 3 165 610 A1**
(43) Date of publication of application: **10.05.2017**
(21) Application number: 15815143.1
(22) Date of filing: 02.07.2015
(51) Int. Cl.: C12P 21/02, C12N 9/00

(54) **METHOD FOR PRODUCING OXIDIZED -GLUTAMYLCYSTEINE AND OXIDIZED GLUTATHIONE**

(30) Priority: 02.07.2014 JP 2014137202
(71) Applicant: Kaneka Corporation, Osaka-shi, Osaka 530-8288 (JP)
(72) Inventor: NOJIRI Masutoshi, Takasago-shi Hyogo 676-8688 (JP); YASOHARA Yoshihiko, Takasago-shi Hyogo 676-8688 (JP)
(74) Representative: Hoffmann Eitle
(86) International application number: PCT/JP2015/069141
(87) International publication number: WO 2016/002884

(57) **Abstract**

The technical problem to be solved by the present invention is to provide a method for producing oxidized glutathione, GSSG and a precursor thereof, i.e., oxidized γ-glutamylcysteine, by a simple process. As a means for solving the problem, the method for producing GSSG according to the present invention comprises step A' of reacting L-cystine and L-glutamic acid to produce oxidized γ-glutamylcysteine and step B' of reacting oxidized γ-glutamylcysteine and glycine to produce GSSG.

### Sequence Listing

SEQUENCE LISTING

<110>  Kaneka Corporation

<120>  A method for producing oxidized gamma-glutamylcysteine and oxidized
glutathione

<130>  B140113

<150>  JP 2014-137202
<151>  2014-07-02

<160>  16

<170>  PatentIn version 3.5

<210>  1
<211>  1557
<212>  DNA
<213>  Escherichia coli

<400>  1
atgatcccgg acgtatcaca ggcgctggcc tggctggaaa aacatcctca ggcgttaaag      60

gggatacagc gtgggctgga gcgcgaaact ttgcgtgtta atgctgatgg cacactggca     120

acaacaggtc atcctgaagc attaggttcc gcactgacgc acaaatggat tactaccgat     180

tttgcggaag cattgctgga attcattaca ccagtggatg gtgatattga acatatgctg     240

acctttatgc gcgatctgca tcgttatacg gcgcgcaata tgggcgatga gcggatgtgg     300

ccgttaagta tgccatgcta catcgcagaa ggtcaggaca tcgaactggc acagtacggc     360

acttctaaca ccggacgctt taaaacgctg tatcgtgaag ggctgaaaaa tcgctacggc     420

gcgctgatgc aaaccatttc cggcgtgcac tacaatttct ctttgccaat ggcattctgg     480

caagcgaagt gcggtgatat ctcgggcgct gatgccaaag agaaaatttc tgcgggctat     540

ttccgcgtta tccgcaatta ctatcgtttc ggttgggtca ttccttatct gtttggtgca     600

tctccggcga tttgttcttc tttcctgcaa ggaaaaccaa cgtcgctgcc gtttgagaaa     660

accgagtgcg gtatgtatta cctgccgtat gcgacctctc ttcgtttgag cgatctcggc     720

tataccaata aatcgcaaag caatcttggt attaccttca acgatcttta cgagtacgta     780

gcgggcctta aacaggcaat caaaacgcca tcggaagagt acgcgaagat tggtattgag     840

aaagacggta agaggctgca aatcaacagc aacgtgttgc agattgaaaa cgaactgtac     900

gcgccgattc gtccaaaacg cgttacccgc agcggcgagt cgccttctga tgcgctgtta     960

cgtggcggca ttgaatatat tgaagtgcgt tcgctggaca tcaacccgtt ctcgccgatt    1020

ggtgtagatg aacagcaggt gcgattcctc gacctgttta tggtctggtg tgcgctggct    1080

gatgcaccgg aaatgagcag tagcgaactt gcctgtacac gcgttaactg gaaccgggtg    1140

atcctcgaag gtcgcaaacc gggtctgacg ctgggtatcg gctgcgaaac cgcacagttc    1200

ccgttaccgc aggtgggtaa agatctgttc cgcgatctga aacgcgtcgc gcaaacgctg    1260

gatagtatta acggcggcga agcgtatcag aaagtgtgtg atgaactggt tgcctgcttc 1320

gataatcccg atctgacttt ctctgcccgt atcttaaggt ctatgattga tactggtatt 1380

ggcggaacag gcaaagcatt tgcagaagcc taccgtaatc tgctgcgtga agagccgctg 1440

gaaattctgc gcgaagagga ttttgtagcc gagcgcgagg cgtctgaacg ccgtcagcag 1500

gaaatggaag ccgctgatac cgaaccgttt gcggtgtggc tggaaaaaca cgcctga 1557

<210> 2
<211> 43
<212> DNA
<213> Artificial

<220>
<223> primer

<400> 2
gcagagctct aaggaggtta caatgatccc ggacgtatca cag 43

<210> 3
<211> 37
<212> DNA
<213> Artificial

<220>
<223> primer

<400> 3
gcgggtacct tatcaggcgt gtttttccag ccacacc 37

<210> 4
<211> 951
<212> DNA
<213> Escherichia coli

<400> 4
atgatcaagc tcggcatcgt gatggacccc atcgcaaaca tcaacatcaa gaaagattcc 60

agttttgcta tgttgctgga agcacagcgt cgtggttacg aacttcacta tatggagatg 120

ggcgatctgt atctgatcaa tggtgaagcc cgcgcccata cccgcacgct gaacgtgaag 180

cagaactacg aagagtggtt ttcgttcgtc ggtgaacagg atctgccgct ggccgatctc 240

gatgtgatcc tgatgcgtaa agacccgccg tttgataccg agtttatcta cgcgacctat 300

attctggaac gtgccgaaga gaaagggacg ctgatcgtta acaagccgca gagcctgcgc 360

gactgtaacg agaaactgtt taccgcctgg ttctctgact taacgccaga aacgctggtt 420

acgcgcaata aagcgcagct aaaagcgttc tgggagaaac acagcgacat cattcttaag 480

ccgctggacg gtatgggcgg cgcgtcgatt ttccgcgtga aagaaggcga tccaaacctc 540

ggcgtgattg ccgaaaccct gactgagcat ggcactcgct actgcatggc gcaaaattac 600

ctgccagcca ttaaagatgg cgacaaacgc gtgctggtgg tggatggcga gccggtaccg 660

tactgcctgg cgcgtattcc gcaggggggc gaaacccgtg gcaatctggc tgccggtggt 720

cgcggtgaac ctcgtccgct gacggaaagt gactggaaaa tcgcccgtca gatcgggccg 780

acgctgaaag aaaaagggct gatttttgtt ggtctggata tcatcggcga ccgtctgact 840

gaaattaacg tcaccagccc aacctgtatt cgtgagattg aagcagagtt tccggtgtcg 900

atcaccggaa tgttaatgga tgccatcgaa gcacgtttac agcagcagta a 951

<210> 5
<211> 36
<212> DNA
<213> Artificial

<220>
<223> primer

<400> 5
agcatcgtac atatgatcaa gctcggcatc gtgatg 36

<210> 6
<211> 36
<212> DNA
<213> Artificial

<220>
<223> primer

<400> 6
gcagaattct cattactgct gctgtaaacg tgcttc 36

<210> 7
<211> 2265
<212> DNA
<213> Streptococcus agalactiae

<400> 7
catatgatta ttgaccgtct gctgcaacgc tcccatagcc atctgccgat cctgcaagcc 60

acctttggtc tggaacgtga atccctgcgc attcatcagc cgacccagcg tgtggcccag 120

acgccgcatc cgaaaaccct gggctctcgc aactatcacc cgtacatcca aacggattat 180

agtgaaccgc agctggaact gattaccccg atcgccaaag actctcagga agcaatccgt 240

tttctgaaag ccatttcaga tgttgcaggt cgctcgatta atcatgacga atatctgtgg 300

ccgctgagta tgccgccaaa agtccgtgaa gaagatattc aaatcgctca gctggaagat 360

gcgttcgaat atgactaccg caaatatctg gaaaaaacct acggcaaact gattcagtcc 420

atctcaggta ttcactataa cctgggcctg ggtcaagaac tgctgacctc gctgtttgaa 480

ctgagccagg cggataacgc cattgacttc cagaatcaac tgtatatgaa actgtctcag 540

aattttctgc gttaccgctg gctgctgacc tatctgtacg gcgccagtcc ggtggcagaa 600

gaagatttcc tggaccagaa actgaacaat ccggtccgtt ccctgcgcaa ctcacatctg 660

ggttatgtga atcacaaaga tattcgtatc tcgtatacca gcctgaaaga ttacgtgaac 720

gacctggaaa atgctgttaa atctggccag ctgattgcgg aaaaagaatt ttatagcccg 780

gttcgtctgc gcggctctaa agcctgccgt aactatctgg aaaaaggtat cacgtacctg 840

gaatttcgca ccttcgatct gaatccgttt agtccgattg gtatcaccca ggaaacggtg 900

gataccgttc acctgtttct gctggcgctg ctgtggattg acagctctag tcacatcgat 960

caggacatta aagaagccaa ccgcctgaat gatctgatcg cactgagcca tccgctggaa 1020

aaactgccga accaggctcc ggtttcagat ctggtcgacg caatgcaatc ggtgattcag 1080

cactttaatc tgagcccgta ttaccaagat ctgctggaaa gtgttaaacg tcagatccaa 1140

tccccggaac tgaccgttgc gggtcagctg ctggaaatga ttgaaggtct gtccctggaa 1200

accttcggcc agcgccaggg tcagatctat catgattacg catgggaagc tccgtatgcg 1260

ctgaaaggct acgaaacgat ggaactgagc acccaactgc tgctgttcga tgttattcag 1320

aaaggtgtga actttgaagt tctggatgaa caagaccagt tcctgaaact gtggcataat 1380

tcccacatcg aatatgtgaa aaacggcaat atgacgtcaa aagataacta catcgttccg 1440

ctggcgatgg ccaataaagt ggttaccaag aaaattctgg acgaaaaaca ctttccgacg 1500

ccgttcggtg atgaatttac cgaccgtaaa gaagcgctga actatttttc gcaaatccag 1560

gataaaccga ttgtcgtgaa accgaaaagc acgaacttcg gcctgggtat ttctatcttt 1620

aaaaccagtg ccaatctggc atcctacgaa aaagctattg atatcgcgtt tacggaagac 1680

agcgcgatcc tggtcgaaga atatattgaa ggcaccgaat accgtttctt tgtgctggag 1740

ggtgattgta tcgcggtcct gctgcgtgtg gccgcaaatg ttgttggtga cggtatccat 1800

accatttccc aactggtgaa actgaaaaac cagaatccgc tgcgtggcta tgatcaccgc 1860

tcaccgctgg aagttatcga actgggtgaa gtcgaacagc tgatgctgga acagcaaggc 1920

tacacggtga actcgatccc gccggaaggt accaaaattg aactgcgtcg caactctaat 1980

atcagtacgg gcggtgatag cattgacgtt accaatacga tggatccgac ctataaacag 2040

ctggcagctg aaatggcaga agctatgggc gcatgggtct gtggtgtgga tctgattatc 2100

ccgaacgcta cgcaggcgta cagcaaagac aagaaaaacg cgacctgcat tgaactgaac 2160

tttaatccgc tgatgtatat gcatacctac tgtcaagaag gtccgggtca gagcatcacg 2220

ccgcgcatcc tggcaaaact gtttccggaa ctgtaatgag aattc 2265

<210> 8
<211> 1440
<212> DNA
<213> Acinetobacter johnsonii

<400> 8
catatggata ccgaaacgat tgcctccgcc gtcctgaacg aagaacaact gtcgctggat 60

ctgattgaag cacaatacgc tctgatgaac acccgcgatc agtcgaatgc gaaaagcctg 120

gtgattctgg tttccggcat cgaactggcc ggcaaaggtg aagcagtcaa acagctgcgc 180

gaatgggtgg acccgcgttt tctgtatgtt aaagccgatc cgccgcacct gtttaatctg 240

aaacagccgt tctggcaacc gtatacccgc tttgttccgg cggagggtca gattatggtc 300

tggttcggca actggtacgg tgatctgctg gctaccgcga tgcacgcatc gaaaccgctg 360

gatgacacgc tgtttgatga atatgtcagc aatatgcgtg cgttcgaaca ggatctgaaa 420

aacaataacg tcgacgtgct gaaagtgtgg tttgatctgt cctggaaatc actgcaaaaa 480

cgcctggatg acatggatcc gtctgaagtt cattggcaca aactgcatgg cctggactgg 540

cgtaacaaaa aacagtacga taccctgcaa aaactgcgta cccgctttac ggatgactgg 600

cagattatcg atggtgaaga tgaagacctg cgcaatcaca acttcgccca agcaattctg 660

acggccctgc gtcattgccc ggaacacgaa aagaaagcgg ccctgaaatg gcagcaagca 720

ccgattccgg acatcctgac ccagtttgaa gtgccgcaag ctgaagatgc gaactacaaa 780

tcagaactga aaaaactgac gaaacaggtt gctgatgcga tgcgttgtga tgaccgcaaa 840

gtggttatcg ccttcgaagg catggacgca gctggtaaag gcggtgcaat taaacgcatc 900

gttaaaaaac tggatccgcg tgaatacgaa attcatacca tcgcggcccc ggaaaaatat 960

gaactgcgtc gcccgtacct gtggcgcttt tggtctaaac tgcagagtga tgacattacc 1020

atcttcgatc gtacgtggta tggccgcgtt ctggtcgaac gtgtcgaagg ttttgcgacc 1080

gaagtggaat ggcagcgcgc ctacgcagaa attaatcgtt ttgagaaaaa cctgagctct 1140

agtcagacgg tgctgattaa attctggctg gctatcgata aagacgaaca agcagctcgt 1200

tttaaagccc gcgaaagcac cccgcataaa cgtttcaaaa tcacggaaga agattggcgt 1260

aatcgcgaca aatgggatga ctatctgaaa gcggccgcag atatgttcgc tcacaccgac 1320

acgtcctatg cgccgtggta cattatctca accaacgata aacagcaagc tcgtatcgaa 1380

gtcctgcgtg ccatcctgaa acaactgaaa gccgaccgtg acaccgacta atgagaattc 1440

<210> 9
<211> 518
<212> PRT
<213> Escherichia coli

<400> 9

Met Ile Pro Asp Val Ser Gln Ala Leu Ala Trp Leu Glu Lys His Pro
1               5                   10                  15

Gln Ala Leu Lys Gly Ile Gln Arg Gly Leu Glu Arg Glu Thr Leu Arg
            20                  25                  30

Val Asn Ala Asp Gly Thr Leu Ala Thr Thr Gly His Pro Glu Ala Leu
        35                  40                  45

Gly Ser Ala Leu Thr His Lys Trp Ile Thr Thr Asp Phe Ala Glu Ala
    50                  55                  60

Leu Leu Glu Phe Ile Thr Pro Val Asp Gly Asp Ile Glu His Met Leu
65                  70                  75                  80

Thr Phe Met Arg Asp Leu His Arg Tyr Thr Ala Arg Asn Met Gly Asp
                85                  90                  95

Glu Arg Met Trp Pro Leu Ser Met Pro Cys Tyr Ile Ala Glu Gly Gln
            100                 105                 110

Asp Ile Glu Leu Ala Gln Tyr Gly Thr Ser Asn Thr Gly Arg Phe Lys
        115                 120                 125

Thr Leu Tyr Arg Glu Gly Leu Lys Asn Arg Tyr Gly Ala Leu Met Gln
    130                 135                 140

Thr Ile Ser Gly Val His Tyr Asn Phe Ser Leu Pro Met Ala Phe Trp
145                 150                 155                 160

Gln Ala Lys Cys Gly Asp Ile Ser Gly Ala Asp Ala Lys Glu Lys Ile
                165                 170                 175

Ser Ala Gly Tyr Phe Arg Val Ile Arg Asn Tyr Tyr Arg Phe Gly Trp
            180                 185                 190

Val Ile Pro Tyr Leu Phe Gly Ala Ser Pro Ala Ile Cys Ser Ser Phe
        195                 200                 205

Leu Gln Gly Lys Pro Thr Ser Leu Pro Phe Glu Lys Thr Glu Cys Gly
    210                 215                 220

Met Tyr Tyr Leu Pro Tyr Ala Thr Ser Leu Arg Leu Ser Asp Leu Gly
225                 230                 235                 240

Tyr Thr Asn Lys Ser Gln Ser Asn Leu Gly Ile Thr Phe Asn Asp Leu
                245                 250                 255

Tyr Glu Tyr Val Ala Gly Leu Lys Gln Ala Ile Lys Thr Pro Ser Glu
            260                 265                 270

Glu Tyr Ala Lys Ile Gly Ile Glu Lys Asp Gly Lys Arg Leu Gln Ile
        275                 280                 285

Asn Ser Asn Val Leu Gln Ile Glu Asn Glu Leu Tyr Ala Pro Ile Arg
    290                 295                 300

Pro Lys Arg Val Thr Arg Ser Gly Glu Ser Pro Ser Asp Ala Leu Leu
305                 310                 315                 320

Arg Gly Gly Ile Glu Tyr Ile Glu Val Arg Ser Leu Asp Ile Asn Pro
                325                 330                 335

Phe Ser Pro Ile Gly Val Asp Glu Gln Gln Val Arg Phe Leu Asp Leu
            340                 345                 350

Phe Met Val Trp Cys Ala Leu Ala Asp Ala Pro Glu Met Ser Ser Ser
        355                 360                 365

Glu Leu Ala Cys Thr Arg Val Asn Trp Asn Arg Val Ile Leu Glu Gly
    370                 375                 380

Arg Lys Pro Gly Leu Thr Leu Gly Ile Gly Cys Glu Thr Ala Gln Phe
385                 390                 395                 400

Pro Leu Pro Gln Val Gly Lys Asp Leu Phe Arg Asp Leu Lys Arg Val
                405                 410                 415

Ala Gln Thr Leu Asp Ser Ile Asn Gly Gly Glu Ala Tyr Gln Lys Val
            420                 425                 430

Cys Asp Glu Leu Val Ala Cys Phe Asp Asn Pro Asp Leu Thr Phe Ser
        435                 440                 445

Ala Arg Ile Leu Arg Ser Met Ile Asp Thr Gly Ile Gly Gly Thr Gly
    450                 455                 460

Lys Ala Phe Ala Glu Ala Tyr Arg Asn Leu Leu Arg Glu Glu Pro Leu
465                 470                 475                 480

Glu Ile Leu Arg Glu Glu Asp Phe Val Ala Glu Arg Glu Ala Ser Glu
                485                 490                 495

Arg Arg Gln Gln Glu Met Glu Ala Ala Asp Thr Glu Pro Phe Ala Val
            500                 505                 510

Trp Leu Glu Lys His Ala
        515

<210>  10
<211>  316

<212>  PRT
<213>  Escherichia coli

<400>  10

<210>  11
<211>  2253
<212>  DNA
<213>  Streptococcus agalactiae

<400>  11
atgattatcg atcgactgtt acaaagaagc cactctcatc taccgattct acaagctaca      60

tttggcttag agagagaaag ccttcgtatt caccaaccca ctcaaagggt tgctcaaaca     120

ccccatccca aaacattagg gagtcgtaac tatcatcctt atatccaaac ggattatagt     180

gaacctcaat tagaactcat tacacctatt gcaaaggata gccaagaagc cattcgattt     240

ttgaaagcta taagtgatgt tgctggacgc tctatcaacc acgatgaata cttatggccg     300

ctttctatgc cacccaaagt gagggaagaa gacatacaga ttgcccaatt agaggatgct     360

tttgaatatg attatcgcaa atatttggag aagacttatg gaaaactaat acaatccatc     420

tctggaattc actataatct cggtttaggt caagagttat taacttctct ctttgaattg     480

agtcaagcag ataatgctat tgattttcag aaccagcttt acatgaaatt gtctcaaaat     540

tttttacgtt accgttggtt actaacttat ctatacggag cgagcccagt agctgaagaa     600

gactttttag atcagaaact gaataaccct gtccggtcac ttagaaatag tcacttaggt     660

tatgtgaatc ataaagatat tcgtatttct tatactagct tgaaggatta tgtcaacgat     720

ctagaaaatg ctgtaaaaag tgggcaattg attgctgaga aagaatttta ttcacctgtt     780

cgtctccgtg gtagtaaggc ctgtcgtaat tatttagaaa aaggaattac atatttagag     840

tttcgcactt ttgatctcaa cccatttagc ccaatcggta taacgcagga aactgttgat     900

actgttcacc ttttcttatt ggcgcttttg tggatagatt ctagtagtca tattgaccaa     960

gatatcaagg aagccaatag attaaatgac cttatagcac ttagtcatcc attagaaaaa    1020

ttacctaatc aagcaccagt ttctgactta gtggatgcta tgcaatctgt tatccaacat    1080

tttaacttat caccttacta tcaagaccta ttggaatctg taaaaaggca aattcagtca 1140

cctgaattaa cggtagctgg tcaactttta gaaatgattg aaggactttc cttagaaaca 1200

tttggacaaa gacaaggaca aatttatcac gattatgctt gggaagctcc ctacgcctta 1260

aagggatatg aaacgatgga actttctacc caattgctac tatttgacgt tatccaaaaa 1320

ggagtcaact tcgaagtgct ggatgaacaa gatcaattcc taaaattatg gcacaatagt 1380

catattgagt acgttaaaaa tggtaatatg acctcaaaag ataactatat tgtaccgctc 1440

gctatggcta acaaagttgt tacaaaaaaa atcctagatg aaaagcattt cccaactcct 1500

tttggagatg aatttactga ccgtaaagag gcacttaact atttttcaca aatccaagat 1560

aaaccaatcg tcgttaagcc aaaatctaca aactttggtt taggcatttc tatttttaaa 1620

acatcagcta acttagcttc ttatgaaaaa gcaattgaca tcgcttttac cgaagatagt 1680

gctattcttg tggaagaata tattgagggt accgaatacc gtttcttcgt ccttgagggg 1740

gactgtattg ctgtattgct tagggtagca gctaatgtcg ttggagatgg tattcatact 1800

attagtcagc tagttaaact taaaaaccaa aaccctctca gaggctatga tcatcgctct 1860

cctctagagg ttattgagct cggagaagta gaacagttaa tgttggaaca acaaggatac 1920

actgttaaca gtatccctcc agaaggaaca aaaatagaac ttcgccgtaa ttctaatatt 1980

tccactggcg gtgactctat agatgtaaca aatacaatgg atcctacata taaacaacta 2040

gcagctgaga tggcagaggc tatgggggct tgggtttgtg gagttgattt aattattcct 2100

aatgccactc aagcatactc aaaagataag aaaaatgcta cttgcattga actaaacttt 2160

aacccattga tgtatatgca cacctactgt caagaggggc ctggtcaatc tattacacct 2220

cgaattttgg ctaaactgtt cccagaatta taa 2253

<210> 12
<211> 750
<212> PRT
<213> Streptococcus agalactiae

<400> 12

Met Ile Ile Asp Arg Leu Leu Gln Arg Ser His Ser His Leu Pro Ile
1               5                   10                  15

Leu Gln Ala Thr Phe Gly Leu Glu Arg Glu Ser Leu Arg Ile His Gln
            20                  25                  30

Pro Thr Gln Arg Val Ala Gln Thr Pro His Pro Lys Thr Leu Gly Ser
        35                  40                  45

Arg Asn Tyr His Pro Tyr Ile Gln Thr Asp Tyr Ser Glu Pro Gln Leu
    50                  55                  60

Glu Leu Ile Thr Pro Ile Ala Lys Asp Ser Gln Glu Ala Ile Arg Phe
65                  70                  75                  80

Leu Lys Ala Ile Ser Asp Val Ala Gly Arg Ser Ile Asn His Asp Glu
                85                  90                  95

Tyr Leu Trp Pro Leu Ser Met Pro Pro Lys Val Arg Glu Glu Asp Ile
            100                 105                 110

Gln Ile Ala Gln Leu Glu Asp Ala Phe Glu Tyr Asp Tyr Arg Lys Tyr
        115                 120                 125

Leu Glu Lys Thr Tyr Gly Lys Leu Ile Gln Ser Ile Ser Gly Ile His
    130                 135                 140

Tyr Asn Leu Gly Leu Gly Gln Glu Leu Leu Thr Ser Leu Phe Glu Leu
145                 150                 155                 160

Ser Gln Ala Asp Asn Ala Ile Asp Phe Gln Asn Gln Leu Tyr Met Lys
                165                 170                 175

Leu Ser Gln Asn Phe Leu Arg Tyr Arg Trp Leu Leu Thr Tyr Leu Tyr
            180                 185                 190

Gly Ala Ser Pro Val Ala Glu Glu Asp Phe Leu Asp Gln Lys Leu Asn
        195                 200                 205

Asn Pro Val Arg Ser Leu Arg Asn Ser His Leu Gly Tyr Val Asn His
    210                 215                 220

Lys Asp Ile Arg Ile Ser Tyr Thr Ser Leu Lys Asp Tyr Val Asn Asp
225                 230                 235                 240

Leu Glu Asn Ala Val Lys Ser Gly Gln Leu Ile Ala Glu Lys Glu Phe
                245                 250                 255

Tyr Ser Pro Val Arg Leu Arg Gly Ser Lys Ala Cys Arg Asn Tyr Leu
            260                 265                 270

Glu Lys Gly Ile Thr Tyr Leu Glu Phe Arg Thr Phe Asp Leu Asn Pro
        275                 280                 285

Phe Ser Pro Ile Gly Ile Thr Gln Glu Thr Val Asp Thr Val His Leu
    290                 295                 300

Phe Leu Leu Ala Leu Leu Trp Ile Asp Ser Ser Ser His Ile Asp Gln

305                 310                 315                 320

Asp Ile Lys Glu Ala Asn Arg Leu Asn Asp Leu Ile Ala Leu Ser His
                325                 330                 335

Pro Leu Glu Lys Leu Pro Asn Gln Ala Pro Val Ser Asp Leu Val Asp
            340                 345                 350

Ala Met Gln Ser Val Ile Gln His Phe Asn Leu Ser Pro Tyr Tyr Gln
        355                 360                 365

Asp Leu Leu Glu Ser Val Lys Arg Gln Ile Gln Ser Pro Glu Leu Thr
    370                 375                 380

Val Ala Gly Gln Leu Leu Glu Met Ile Glu Gly Leu Ser Leu Glu Thr
385                 390                 395                 400

Phe Gly Gln Arg Gln Gly Gln Ile Tyr His Asp Tyr Ala Trp Glu Ala
                405                 410                 415

Pro Tyr Ala Leu Lys Gly Tyr Glu Thr Met Glu Leu Ser Thr Gln Leu
            420                 425                 430

Leu Leu Phe Asp Val Ile Gln Lys Gly Val Asn Phe Glu Val Leu Asp
        435                 440                 445

Glu Gln Asp Gln Phe Leu Lys Leu Trp His Asn Ser His Ile Glu Tyr
    450                 455                 460

Val Lys Asn Gly Asn Met Thr Ser Lys Asp Asn Tyr Ile Val Pro Leu
465                 470                 475                 480

Ala Met Ala Asn Lys Val Val Thr Lys Lys Ile Leu Asp Glu Lys His
                485                 490                 495

Phe Pro Thr Pro Phe Gly Asp Glu Phe Thr Asp Arg Lys Glu Ala Leu
            500                 505                 510

Asn Tyr Phe Ser Gln Ile Gln Asp Lys Pro Ile Val Val Lys Pro Lys
        515                 520                 525

Ser Thr Asn Phe Gly Leu Gly Ile Ser Ile Phe Lys Thr Ser Ala Asn
    530                 535                 540

Leu Ala Ser Tyr Glu Lys Ala Ile Asp Ile Ala Phe Thr Glu Asp Ser
545                 550                 555                 560

Ala Ile Leu Val Glu Glu Tyr Ile Glu Gly Thr Glu Tyr Arg Phe Phe
                565                 570                 575

Val Leu Glu Gly Asp Cys Ile Ala Val Leu Leu Arg Val Ala Ala Asn
            580                 585                 590

Val Val Gly Asp Gly Ile His Thr Ile Ser Gln Leu Val Lys Leu Lys
        595                 600                 605

Asn Gln Asn Pro Leu Arg Gly Tyr Asp His Arg Ser Pro Leu Glu Val
    610                 615                 620

Ile Glu Leu Gly Glu Val Glu Gln Leu Met Leu Glu Gln Gln Gly Tyr
625                 630                 635                 640

Thr Val Asn Ser Ile Pro Pro Glu Gly Thr Lys Ile Glu Leu Arg Arg
                645                 650                 655

Asn Ser Asn Ile Ser Thr Gly Gly Asp Ser Ile Asp Val Thr Asn Thr
            660                 665                 670

Met Asp Pro Thr Tyr Lys Gln Leu Ala Ala Glu Met Ala Glu Ala Met
        675                 680                 685

Gly Ala Trp Val Cys Gly Val Asp Leu Ile Ile Pro Asn Ala Thr Gln
    690                 695                 700

Ala Tyr Ser Lys Asp Lys Lys Asn Ala Thr Cys Ile Glu Leu Asn Phe
705                 710                 715                 720

Asn Pro Leu Met Tyr Met His Thr Tyr Cys Gln Glu Gly Pro Gly Gln
                725                 730                 735

Ser Ile Thr Pro Arg Ile Leu Ala Lys Leu Phe Pro Glu Leu
            740                 745                 750

<210>  13
<211>  645
<212>  DNA
<213>  Escherichia coli

<400>  13
atgcgtatca ttctgcttgg cgctccgggc gcggggaaag ggactcaggc tcagttcatc      60

atggagaaat atggtattcc gcaaatctcc actggcgata tgctgcgtgc tgcggtcaaa     120

tctggctccg agctgggtaa acaagcaaaa gacattatgg atgctggcaa actggtcacc     180

gacgaactgg tgatcgcgct ggttaaagag cgcattgctc aggaagactg ccgtaatggt     240

ttcctgttgg acggcttccc gcgtaccatt ccgcaggcag acgcgatgaa agaagcgggc     300

atcaatgttg attacgttct ggaattcgac gtaccggacg aactgatcgt tgaccgtatc 360

gtcggtcgcc gcgttcatgc gccgtctggt cgtgtttatc acgttaaatt caatccgccg 420

aaagtagaag gcaaagacga cgttaccggt gaagaactga ctacccgtaa agatgatcag 480

gaagagaccg tacgtaaacg tctggttgaa taccatcaga tgacagcacc gctgatcggc 540

tactactcca aagaagcaga agcgggtaat accaaatacg cgaaagttga cggcaccaag 600

ccggttgctg aagttcgcgc tgatctggaa aaaatcctcg gctaa 645

<210> 14
<211> 214
<212> PRT
<213> Escherichia coli

<400> 14

Met Arg Ile Ile Leu Leu Gly Ala Pro Gly Ala Gly Lys Gly Thr Gln
1               5                   10                  15

Ala Gln Phe Ile Met Glu Lys Tyr Gly Ile Pro Gln Ile Ser Thr Gly
            20                  25                  30

Asp Met Leu Arg Ala Ala Val Lys Ser Gly Ser Glu Leu Gly Lys Gln
        35                  40                  45

Ala Lys Asp Ile Met Asp Ala Gly Lys Leu Val Thr Asp Glu Leu Val
    50                  55                  60

Ile Ala Leu Val Lys Glu Arg Ile Ala Gln Glu Asp Cys Arg Asn Gly
65                  70                  75                  80

Phe Leu Leu Asp Gly Phe Pro Arg Thr Ile Pro Gln Ala Asp Ala Met
                85                  90                  95

Lys Glu Ala Gly Ile Asn Val Asp Tyr Val Leu Glu Phe Asp Val Pro
            100                 105                 110

Asp Glu Leu Ile Val Asp Arg Ile Val Gly Arg Arg Val His Ala Pro
        115                 120                 125

Ser Gly Arg Val Tyr His Val Lys Phe Asn Pro Pro Lys Val Glu Gly
    130                 135                 140

Lys Asp Asp Val Thr Gly Glu Glu Leu Thr Thr Arg Lys Asp Asp Gln
145                 150                 155                 160

Glu Glu Thr Val Arg Lys Arg Leu Val Glu Tyr His Gln Met Thr Ala
                165                 170                 175

Pro Leu Ile Gly Tyr Tyr Ser Lys Glu Ala Glu Ala Gly Asn Thr Lys
            180                 185                 190

Tyr Ala Lys Val Asp Gly Thr Lys Pro Val Ala Glu Val Arg Ala Asp
        195                 200                 205

Leu Glu Lys Ile Leu Gly
    210

<210>  15
<211>  1428
<212>  DNA
<213>  Acinetobacter johnsonii

<400>  15
atggatacag aaacgatcgc cagtgcagtg ctgaatgaag aacagctttc actggactta      60

attgaagcgc aatatgcgtt gatgaatacc cgtgatcaga gcaatgcaaa aagtttagtg     120

attttggtca gtggaatcga acttgcgggt aaaggcgaag cggtgaaaca gctccgcgaa     180

tgggtcgatc ctcgtttttt atatgtcaaa gccgatccac cgcatctgtt taatctaaaa     240

cagccttttt ggcagcccta tacccgattt gtgcctgccg aagggcaaat tatggtgtgg     300

tttggtaatt ggtatgggga tttgttggct acggccatgc atgcttcaaa gcctttagat     360

gacactttgt ttgatgaata cgtcagcaat atgcgggctt ttgaacagga cttaaaaaat     420

aacaacgtag atgtcttaaa agtttggttc gatttgtcgt ggaagtctct gcaaaagcgt     480

ctagatgata tggacccgag cgaagtgcat tggcataagt tgcatgggct agactggcgc     540

aataaaaaac aatatgacac cttacaaaag ctacgtacgc gcttcaccga tgactggcaa     600

atcattgatg gtgaagatga ggatttgcgt aatcacaatt ttgcacaagc aattttaacg     660

gcactacgac actgcccaga gcatgaaaaa aaggccgcgc taaaatggca gcaagcacca     720

ataccagata ttctgactca gtttgaagtc cctcaagctg aggatgcgaa ctataaatca     780

gaattgaaaa aactcaccaa acaagtggcc gatgccatgc gctgtgatga ccgtaaagtg     840

gtgattgctt ttgaaggtat ggatgctgcg ggtaaagggg gggcgattaa gcgtattgtg     900

aaaaagctcg acccacgaga atatgaaatt cataccattg ccgcacctga aaaatatgag     960

ttacgccgtc cttatctgtg gcgtttttgg agcaaattgc agtcggatga catcactatt    1020

tttgatcgga cgtggtatgg acgcgtttta gtcgagcggg tagaaggctt cgcaaccgag    1080

gtagagtggc aacgcgctta tgcggaaatc aatcgttttg aaaaaaacct cagtagcagc    1140

caaaccgtgc tgattaagtt ttggctggcg attgataaag atgaacaagc agcgcgtttt    1200

aaagcccgcg aaagtactcc gcataaacgt tttaaaatta ccgaagaaga ttggcgcaat    1260

cgcgacaaat gggatgacta tttaaaggca gccgcggata tgtttgcgca taccgacacc    1320

agctatgcgc cttggtatat tatttccacc aatgataaac aacaggcccg cattgaagtc 1380

ttaagggcaa ttttaaaaca gctcaaagcg gatcgcgaca cggattaa 1428

<210> 16
<211> 475
<212> PRT
<213> Acinetobacter johnsonii

<400> 16

Met Asp Thr Glu Thr Ile Ala Ser Ala Val Leu Asn Glu Glu Gln Leu
1               5                   10                  15

Ser Leu Asp Leu Ile Glu Ala Gln Tyr Ala Leu Met Asn Thr Arg Asp
            20                  25                  30

Gln Ser Asn Ala Lys Ser Leu Val Ile Leu Val Ser Gly Ile Glu Leu
        35                  40                  45

Ala Gly Lys Gly Glu Ala Val Lys Gln Leu Arg Glu Trp Val Asp Pro
    50                  55                  60

Arg Phe Leu Tyr Val Lys Ala Asp Pro Pro His Leu Phe Asn Leu Lys
65                  70                  75                  80

Gln Pro Phe Trp Gln Pro Tyr Thr Arg Phe Val Pro Ala Glu Gly Gln
                85                  90                  95

Ile Met Val Trp Phe Gly Asn Trp Tyr Gly Asp Leu Leu Ala Thr Ala
            100                 105                 110

Met His Ala Ser Lys Pro Leu Asp Asp Thr Leu Phe Asp Glu Tyr Val
        115                 120                 125

Ser Asn Met Arg Ala Phe Glu Gln Asp Leu Lys Asn Asn Asn Val Asp
    130                 135                 140

Val Leu Lys Val Trp Phe Asp Leu Ser Trp Lys Ser Leu Gln Lys Arg
145                 150                 155                 160

Leu Asp Asp Met Asp Pro Ser Glu Val His Trp His Lys Leu His Gly
                165                 170                 175

Leu Asp Trp Arg Asn Lys Lys Gln Tyr Asp Thr Leu Gln Lys Leu Arg
            180                 185                 190

Thr Arg Phe Thr Asp Asp Trp Gln Ile Ile Asp Gly Glu Asp Glu Asp
        195                 200                 205

Leu Arg Asn His Asn Phe Ala Gln Ala Ile Leu Thr Ala Leu Arg His
    210                 215                 220

Cys Pro Glu His Glu Lys Lys Ala Ala Leu Lys Trp Gln Gln Ala Pro
225                 230                 235                 240

Ile Pro Asp Ile Leu Thr Gln Phe Glu Val Pro Gln Ala Glu Asp Ala
                245                 250                 255

Asn Tyr Lys Ser Glu Leu Lys Lys Leu Thr Lys Gln Val Ala Asp Ala
            260                 265                 270

Met Arg Cys Asp Asp Arg Lys Val Val Ile Ala Phe Glu Gly Met Asp
        275                 280                 285

Ala Ala Gly Lys Gly Gly Ala Ile Lys Arg Ile Val Lys Lys Leu Asp
    290                 295                 300

Pro Arg Glu Tyr Glu Ile His Thr Ile Ala Ala Pro Glu Lys Tyr Glu
305                 310                 315                 320

Leu Arg Arg Pro Tyr Leu Trp Arg Phe Trp Ser Lys Leu Gln Ser Asp
                325                 330                 335

Asp Ile Thr Ile Phe Asp Arg Thr Trp Tyr Gly Arg Val Leu Val Glu
            340                 345                 350

Arg Val Glu Gly Phe Ala Thr Glu Val Glu Trp Gln Arg Ala Tyr Ala
        355                 360                 365

Glu Ile Asn Arg Phe Glu Lys Asn Leu Ser Ser Ser Gln Thr Val Leu
    370                 375                 380

Ile Lys Phe Trp Leu Ala Ile Asp Lys Asp Glu Gln Ala Ala Arg Phe
385                 390                 395                 400

Lys Ala Arg Glu Ser Thr Pro His Lys Arg Phe Lys Ile Thr Glu Glu
                405                 410                 415

Asp Trp Arg Asn Arg Asp Lys Trp Asp Asp Tyr Leu Lys Ala Ala Ala
            420                 425                 430

Asp Met Phe Ala His Thr Asp Thr Ser Tyr Ala Pro Trp Tyr Ile Ile
        435                 440                 445

Ser Thr Asn Asp Lys Gln Gln Ala Arg Ile Glu Val Leu Arg Ala Ile

450                 455                 460

Leu Lys Gln Leu Lys Ala Asp Arg Asp Thr Asp
465                 470                 475

## Description

### Technical Field

The present invention relates to a method for producing oxidized γ-glutamylcysteine.

The present invention also relates to a method for producing oxidized glutathione.

### Background Art

Glutathione is a peptide consisting of three amino acids, i.e., L-cysteine, L-glutamic acid and glycine and present in a wide variety of living organisms including not only humans but also other animals, plants and microorganisms, and is an important compound for living organisms involved in, e.g., removal of reactive oxygen species, detoxification and amino acid metabolism.

Glutathione is present *in vivo* in the form of either one of reduced glutathione (N-(N-γ-L-glutamyl-L-cysteinyl)glycine, in which the thiol group of the L-cysteine residue is reduced in the form of SH (hereinafter will be sometimes referred to as "GSH") and oxidized glutathione in which the thiol group of the L-cysteine residue is oxidized to form a disulfide bond between two glutathione molecules (hereinafter will be sometimes referred to as "GSSG").

It is known that GSSG has higher preservation stability than GSH (e.g., Patent Literature 1). Glutathione is often present in the form of GSH (reduced glutathione) *in vivo* in most cases. GSH is easily produced on a large scale by using e.g., a yeast; however, GSSG has been not yet easily produced on a large scale with high efficiency. In conventional (general) methods, GSH is produced first and then GSH is chemically or biologically oxidized to produce GSSG. For example, in the method disclosed in Patent Literature 1, a yeast extract containing GSH is first produced; and then, while controlling the pH value of the yeast extract to be 6 or more to less than 11, GSH is converted into GSSG in the presence of dissolved oxygen.

### Citation List

### Patent Literatures

Patent Literature 1: JP Patent No. 3160335
Patent Literature 2: JP Patent Publication (Kohyo) No. 2013-505712
Patent Literature 3: JP Patent Publication (Kokai) No. 2013-188177

### Summary of Invention

### Technical Problem

The technical problem to be solved by the present invention is to provide a method for producing GSSG and a precursor compound thereof by a simple process.

### Solution to Problem

The present inventors found that GSSG and a precursor thereof, i.e., oxidized γ-glutamylcysteine, can be produced from relatively inexpensive starting material, L-cystine, by a simple process and reached completion of the present invention. L-cystine is a compound consisting of two L-cysteine molecules bound via a disulfide bond by oxidation of the thiol groups of them. It is known that L-cystine is used, for example, as a starting material for producing L-cysteine (Patent Literature 2) and as a starting material for producing GSH through lactic fermentation (Patent Literature 3); however, a method of producing GSSG directly from L-cystine has not yet been present.

More specifically, the present invention includes the following invention.
(1) A method for producing oxidized γ-glutamylcysteine, characterized by comprising step A of reacting L-cystine and L-glutamic acid in the presence of at least one enzyme selected from the group consisting of γ-glutamylcysteine synthetase and a bifunctional glutathione synthetase, and adenosine triphosphate (ATP, also referred to as adenosine 5'-triphosphate) to produce oxidized γ-glutamylcysteine.
   According to the present invention, oxidized γ-glutamylcysteine can be directly produced from relatively inexpensive L-cystine.
(2) The method according to (1), in which the step A is carried out in conjugation with an ATP regeneration reaction for regenerating adenosine diphosphate (ADP, also referred to as adenosine 5'-diphosphate) into ATP.
   In the step A, ATP is consumed to produce ADP; however, the step A according to the present invention can be continuously performed while regenerating ATP.
(3) The method according to (1) or (2), in which the γ-glutamylcysteine synthetase is derived from *Escherichia coli.*
   Such γ-glutamylcysteine synthetase is preferable since it has a particularly high activity to produce oxidized γ-glutamylcysteine from L-cystine and L-glutamic acid.
(4) The method according to (1) or (2), in which the bifunctional glutathione synthetase is derived from *Streptococcus agalactiae.*
   Such a bifunctional glutathione synthetase is preferable since it has a particularly high activity to produce oxidized γ-glutamylcysteine from L-cystine and L-glutamic acid.
(5) A method for producing oxidized glutathione, characterized by comprising step B of reacting oxidized γ-glutamylcysteine and glycine in the presence of a glutathione synthetase and adenosine triphosphate (ATP) to produce oxidized glutathione.
   According to the present invention, oxidized glutathione can be produced by a simple process.
(6) The method according to (5), in which the step B is carried out in conjugation with an ATP regeneration reaction for regenerating adenosine diphosphate (ADP) into ATP.
   In the step B, ATP is consumed to produce ADP; however, the step B according to the present invention can be continuously performed while regenerating ATP.
(7) The method according to (5) or (6), in which the glutathione synthetase is derived from *Escherichia coli.*
   Such a glutathione synthetase is preferable since it has a particularly high activity to produce oxidized glutathione from oxidized γ-glutamylcysteine and glycine.
(8) The method according to any one of (5) to (7), further having step A of reacting L-cystine and L-glutamic acid in the presence of at least one enzyme selected from the group consisting of γ-glutamylcysteine synthetase and a bifunctional glutathione synthetase, and adenosine triphosphate(ATP) to produce oxidized γ-glutamylcysteine, in which oxidized γ-glutamylcysteine used in the step B is produced by the step A.
   According to the present invention, oxidized glutathione can be produced from relatively inexpensive L-cystine by a simple process.
(9) The method according to (8), in which the step A is carried out in conjugation with an ATP regeneration reaction for regenerating adenosine diphosphate (ADP) into ATP.
   In the step A, ATP is consumed to produce ADP; however, the step A according to the present invention can be continuously carried out while regenerating ATP.
(10) The method according to (8) or (9), in which the γ-glutamylcysteine synthetase is derived from *Escherichia coli.*
   Such γ-glutamylcysteine synthetase is preferable since it has a particularly high activity to produce oxidized γ-glutamylcysteine from L-cystine and L-glutamic acid.
(11) The method according to (8) or (9), in which the bifunctional glutathione synthetase is derived from *Streptococcus agalactiae.*
   Such a bifunctional glutathione synthetase is preferable since it has a particularly high activity to produce oxidized γ-glutamylcysteine from L-cystine and L-glutamic acid.
(12) A method for producing oxidized γ-glutamylcysteine characterized by comprising step A' of reacting L-cystine and L-glutamic acid to produce oxidized γ-glutamylcysteine.
   According to the present invention, oxidized γ-glutamylcysteine can be directly produced from relatively inexpensive L-cystine. In the present invention, it is more preferable that the step A' is the step A according to (1).
(13) A method for producing oxidized glutathione, characterized by comprising step B' of reacting oxidized γ-glutamylcysteine and glycine to produce oxidized glutathione.
   According to the present invention, oxidized glutathione can be produced by a simple process. In the present invention, it is more preferable that the step B' is the step B according to (5).
(14) The method according to (13), further having step A' of reacting L-cystine and L-glutamic acid to produce oxidized γ-glutamylcysteine, in which oxidized γ-glutamylcysteine used in the step B' is produced by the step A'.

According to the present invention, oxidized glutathione can be produced from relatively inexpensive L-cystine by a simple process. In the present invention, it is more preferable that the step A' is the step A according to (1).

In the specification, the compounds represented by terms: e.g., "L-cystine", "L-glutamic acid", "glycine ""oxidized γ-glutamylcysteine", "oxidized glutathione", "L-cysteine", "glutamylcysteine", "glutathione", "adenosine triphosphate", "adenosine diphosphate", "adenosine monophosphate", "polyphosphoric acid" and "condensed phosphoric acid" may take any forms including free form, a salt (e.g., a sodium salt and a potassium salt), a solvate (e.g., a hydrate), and an ion.

Oxidized γ-glutamylcysteine in its free form is represented by the following formula:

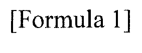

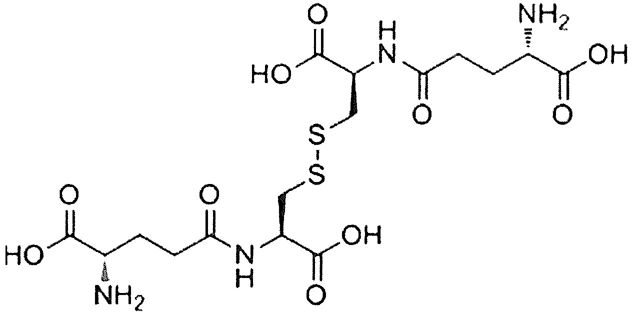

Oxidized glutathione in its free form is represented by the following formula:

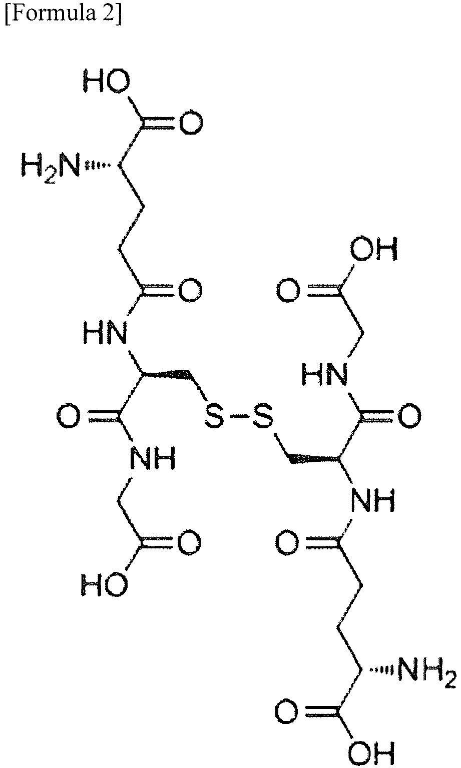

The teachings of Japanese Patent Application No. 2014-137202, on which the priority of the present application is based, are incorporated into this specification.

### Advantageous Effects of Invention

According to the present invention, it is possible to produce oxidized glutathione (GSSG) and oxidized γ-glutamylcysteine from an inexpensive material by a simple process.

### Description of Embodiments

### <Enzymes used in the present invention>

As used herein, γ-glutamylcysteine synthetase will be sometimes referred to simply as "GSH I", glutathione synthetase as "GSH II", a bifunctional glutathione synthetase as "GSH F", adenylate kinase as "ADK", and polyphosphate-dependent AMP transferase as "PAP".

### <GSH I>

The γ-glutamylcysteine synthetase (GSH I) used in the present invention is an enzyme having an activity to recognize L-Cys as a substrate and to catalyze a reaction for producing γ-Glu-Cys by mediating binding the L-Cys to L-Glu in the presence of ATP. GSH I may be any enzyme having this activity. The source, structure and other characteristics of GSH I are not limited. In the present invention, this activity is referred to as γ-glutamylcysteine synthetase activity. One unit (1 U) of the activity means the activity to produce 1 µlmol of γ-glutamylcysteine at 30°C for one minute and is measured in the following measurement condition. The present invention is based on a new finding that γ-glutamylcysteine synthetase also has an activity to produce oxidized γ-glutamylcysteine by reacting L-cystine and L-glutamic acid in the presence of ATP.

### (Measurement condition)

The reaction comprises adding an enzyme solution to a 50 mM tris hydrochloride buffer solution (pH8.0) containing 10 mM ATP, 15 mM L-glutamic acid, 15 mM L-cysteine and 10 mM magnesium sulfate and then keeping the reaction solution at 30°C. The reaction is terminated by adding 6N hydrochloric acid. The γ-glutamylcysteine in the reaction solution is quantified by high-performance liquid chromatography.

The conditions for the high-performance liquid chromatography are as follows. In the conditions, reduced glutathione (GSH), reduced γ-glutamylcysteine (γ-GC), oxidized γ-GC and oxidized glutathione (GSSG) sequentially elute in this order.

### [HPLC conditions]

Column: ODS-HG-3 (4.6 mmϕ x 150 mm, manufactured by Nomura Chemical Co., Ltd.);
Eluent: Solution prepared by dissolving potassium dihydrogenphosphate (12.2 g) and sodium heptanesulfonate (3.6 g) with distilled water (1.8 L), controlling the pH of the solution with phosphoric acid to be pH2.8, adding methanol (186 ml) and dissolving it;
Flow rate: 1.0 ml/minute;
Column temperature: 40°C;
Measurement wavelength: 210 nm.

GSH I having a γ-glutamylcysteine synthetase activity (specific activity) of 0.5 U or more per 1 mg of protein is preferably used as GSH I.

The source from which GSH I is derived is not limited and GSH I derived from e.g., microorganisms, animals and plants can be used. GSH I derived from a microorganism is preferable, particularly GSH I derived from an intestinal microbe, such as *Escherichia coli,* a corynebacterium and an eukaryotic microorganism, such as a yeast, are preferable.

The nucleotide sequence of GSH I derived from *Escherichia coli* and the amino acid sequence encoded by the nucleotide sequence are specifically represented by SEQ ID NO: 1 and SEQ ID NO: 9, respectively.

GSH I that can be used includes not only GSH I consisting of the amino acid sequence represented by SEQ ID NO: 9 but also other polypeptides having GSH I activity, such as an active mutant thereof and orthologue from other species. Said other polypeptides having GSH I activity include a polypeptide exhibiting an activity of preferably 10% or more, preferably 40% or more, more preferably 60% or more, more preferably 80% or more and further preferably 90% or more relative to the activity of the GSH I consisting of the amino acid sequence represented by SEQ ID NO: 9 in the aforementioned activity measurement conditions; and preferably, a polypeptide exhibiting an activity of preferably 10% or more, preferably 40% or more, more preferably 60% or more, more preferably 80% or more and further preferably 90% or more relative to the activity to produce oxidized γ-glutamylcysteine from L-cystine and L-glutamic acid in the presence of ATP of the GSH I consisting of the amino acid sequence represented by SEQ ID NO: 9. Examples of said other polypeptides having GSH I activity, such as an active mutant thereof and orthologue from other species, include a polypeptide consisting of an amino acid sequence having addition, deletion or substitution of one or several amino acids in the amino acid sequence represented by SEQ ID NO: 9 (particularly preferably, a polypeptide consisting of an amino acid sequence having substitution, deletion and/or addition, preferably deletion and/or addition of one or several amino acids in total at either one or both of the N terminal and the C terminal of the amino acid sequence represented by SEQ ID NO: 9); and a polypeptide consisting of an amino acid sequence having an amino acid identity of 80% or more, preferably 85% or more, more preferably 90% or more, 95% or more, 97% or more, 98% or more or 99% or more with the amino acid sequence represented by SEQ ID NO: 9. Further examples of said other polypeptides that can be used herein include a GSH I-active fragment of at least one polypeptide selected from the group consisting of the polypeptide consisting of the amino acid sequence represented by SEQ ID NO: 9; a polypeptide consisting of an amino acid sequence having addition, deletion or substitution of one or several amino acids in the amino acid sequence represented by SEQ ID NO: 9; and a polypeptide consisting of the amino acid sequence having the aforementioned amino acid identity with the amino acid sequence represented by SEQ ID NO: 9. The fragment may be a polypeptide consisting of amino acids of preferably 250 or more, more preferably 300 or more, more preferably 400 or more and more preferably 500 or more. The fragment also has an activity to produce oxidized γ-glutamylcysteine from L-cystine and L-glutamic acid in the presence of ATP. In the specification, "several" refers to, for example, 2 to 20, 2 to 15, 2 to 10, 2 to 7, 2 to 5, 2 to 4 or 2 to 3. The "amino acid identity" refers to the percentage (%) of the number of identical amino acid residues relative to the total number of amino acid residues of the protein represented by SEQ ID NO: 9, when two amino acid sequences are aligned so as to obtain a highest degree of matching of amino acids between the two sequences, if necessary by introducing a gap. The amino acid identity can be calculated by using a protein search system, such as BLAST and FASTA (Karlin, S. et al., 1993, Proc. Natl. Acad. Sci. USA, 90: 5873-5877; Altschul, S. F. et al., 1990, J. Mol. Biol., 215: 403-410; Pearson, W.R. et al., 1988, Proc. Natl. Acad. Sci. USA, 85: 2444-2448). Furthermore, the substitution of amino acids is made preferably in accordance with conservative amino acid substitution. The "conservative amino acid substitution" refers to substitution between amino acids having analogous properties with respect to e.g., charge, side chain, polarity and aromaticity. The amino acids analogous in property are categorized as follows: a basic amino acid (arginine, lysine, histidine); an acidic amino acid (aspartic acid, glutamic acid); an uncharged polar amino acid (glycine, asparagine, glutamine, serine, threonine, cysteine, tyrosine); a nonpolar amino acid (leucine, isoleucine, alanine, valine, proline, phenylalanine, tryptophan, methionine); a branched-chain amino acid (leucine, valine, isoleucine); and an aromatic amino acid (phenylalanine, tyrosine, tryptophan, histidine). The aforementioned polypeptides each may be chemically modified.

The nucleotide sequence of a gene (DNA or RNA) encoding GSH I that can be used for preparation of GSH I is not limited to the nucleotide sequence represented by SEQ ID NO: 1. The nucleotide sequence may be any nucleotide sequence encoding the amino acid sequence of a desired GSH I and being suited to the host organism.

### <GSH II>

The glutathione synthetase (GSH II) used in the present invention is an enzyme having an activity to recognize γ-Glu-Cys as a substrate and to catalyze a reaction for producing γ-Glu-Cys-Gly by mediating binding the γ-Glu-Cys to Gly in the presence of ATP. GSH II may be any enzyme having this activity. The source, structure and other characteristics of GSH II are not limited. In the present invention, the activity is referred to as glutathione synthetase activity. One unit (1 U) of the activity means the activity to produce 1 µmol of glutathione at 30°C for one minute and is measured in the following measurement conditions. The present invention is based on a new finding that glutathione synthetase also has an activity to produce oxidized glutathione by reacting oxidized γ-glutamylcysteine and glycine in the presence of ATP.

### (Measurement condition)

The reaction comprises adding an enzyme solution to a 50 mM tris hydrochloride buffer solution (pH8.0) containing 10 mM ATP, 15 mM γ-glutamylcysteine, 15 mM glycine and 10 mM magnesium sulfate and then keeping the reaction solution at 30°C. The reaction is terminated by adding 6N hydrochloric acid. The glutathione in the reaction solution is quantified by high-performance liquid chromatography.

The same conditions for the high-performance liquid chromatography as mentioned in the above GSH I activity measurement method are used.

GSH II having a glutathione synthetase activity (specific activity) of 0.5 U or more per 1 mg of protein is preferably used as GSH II.

The source from which GSH II is derived is not limited and GSH II derived from e.g., microorganisms, animals and plants can be used. GSH II derived from a microorganism is preferable, particularly GSH II derived from an intestinal microbe, such as *Escherichia coli,* a corynebacterium and an eukaryotic microorganism, such as yeasts, are preferable.

The nucleotide sequence of GSH II derived from *Escherichia coli* and the amino acid sequence encoded by the nucleotide sequence are specifically represented by SEQ ID NO: 4 and SEQ ID NO: 10, respectively.

GSH II that can be used includes not only GSH II consisting of the amino acid sequence represented by SEQ ID NO: 10 but also other polypeptides having GSH II activity, such as an active mutant thereof and orthologue from other species. Said other polypeptides having GSH II activity include a polypeptide exhibiting an activity of preferably 10% or more, preferably 40% or more, more preferably 60% or more, more preferably 80% or more and further preferably 90% or more relative to the activity of the GSH II consisting of the amino acid sequence represented by SEQ ID NO: 10 in the aforementioned activity measurement conditions; and preferably, a polypeptide exhibiting an activity of preferably 10% or more, preferably 40% or more, more preferably 60% or more, more preferably 80% or more, and further preferably 90% or more relative to the activity to produce oxidized glutathione from oxidized γ-glutamylcysteine and glycine in the presence of ATP of the GSH II consisting of the amino acid sequence represented by SEQ ID NO: 10. Examples of said other polypeptides having GSH II activity, such as an active mutant thereof and orthologue from other species, include a polypeptide consisting of an amino acid sequence having addition, deletion or substitution of one or several amino acids in the amino acid sequence represented by SEQ ID NO: 10 (particularly preferably, a polypeptide consisting of an amino acid sequence having substitution, deletion and/or addition, preferably deletion and/or addition of one or several amino acids in total at either one or both of the N terminal and the C terminal of the amino acid sequence represented by SEQ ID NO: 10); and a polypeptide consisting of an amino acid sequence having an amino acid identity of 80% or more, preferably 85% or more, more preferably 90% or more, 95% or more, 97% or more, 98% or more or 99% or more with the amino acid sequence represented by SEQ ID NO:10. Further examples of said other polypeptides that can be used herein include a GSH II-active fragment of at least one polypeptide selected from the group consisting of the polypeptide consisting of the amino acid sequence represented by SEQ ID NO: 10; a polypeptide consisting of an amino acid sequence having addition, deletion or substitution of one or several amino acids in the amino acid sequence represented by SEQ ID NO: 10; and a polypeptide consisting of the amino acid sequence having the aforementioned amino acid identity with the amino acid sequence represented by SEQ ID NO: 10. The fragment may be a polypeptide consisting of amino acids of preferably 150 or more, more preferably 200 or more, more preferably 300 or more. The fragment also has an activity to produce oxidized glutathione from oxidized γ-glutamylcysteine and glycine in the presence of ATP. The "amino acid identity" refers to the percentage (%) of the number of identical amino acid residues relative to the total number of amino acid residues of the protein represented by SEQ ID NO: 10, when two amino acid sequences are aligned and arranged so as to obtain a highest degree of matching of amino acids between the two sequences, if necessary by introducing a gap. Furthermore, the substitution of amino acids is made preferably in accordance with conservative amino acid substitution. The preferable range of "several", method for calculating amino acid identity and conservative amino acid substitution mentioned herein are the same as described with respect to GSH I. The aforementioned polypeptides each may be chemically modified.

The nucleotide sequence of a gene (DNA or RNA) encoding the GSH II that can be used for preparation of GSH II is not limited to the nucleotide sequence represented by SEQ ID NO: 4. The nucleotide sequence may be any nucleotide sequence encoding the amino acid sequence of a desired GSH II and being suited to the host organism.

### <GSH F>

The bifunctional glutathione synthetase (GSH F) used in the present invention is an enzyme having two activities in combination, one of which is an activity to recognize L-Cys as a substrate and to catalyze a reaction for producing γ-Glu-Cys by mediating binding the L-Cys to L-Glu in the presence of ATP, and the other of which is an activity to recognize γ-Glu-Cys as a substrate and to catalyze a reaction for producing γ-Glu-Cys-Gly by mediating binding the γ-Glu-Cys to Gly in the presence of ATP. GSH F may be any enzyme having these two activities in combination. The source, structure and other characteristics of GSH F are not limited. In the present invention, the combination of the activities is referred to as bifunctional glutathione synthetase activity. One unit (1 U) of the activity means the activity to produce 1 µmol of γ-Glu-Cys-Gly (glutathione) at 30°C for one minute and is measured in the following measurement conditions. The present invention is based on a new finding that bifunctional glutathione synthetase also has an activity to produce oxidized γ-glutamylcysteine by reacting L-cystine and L-glutamic acid in the presence of ATP.

### (Measurement condition)

The reaction comprises adding an enzyme solution to a 50 mM tris hydrochloride buffer solution (pH8.0) containing 10 mM ATP, 15 mM L-glutamic acid, 15 mM L-cysteine, 15 mM glycine and 10 mM magnesium sulfate and then keeping the reaction solution at 30°C. The reaction is terminated by adding 6N hydrochloric acid. The glutathione in the reaction solution is quantified by high-performance liquid chromatography.

The same conditions for the high-performance liquid chromatography as mentioned in GSH I activity measurement method are used.

GSH F having a glutathione synthetase activity (specific activity) of 0.5 U or more per 1 mg of protein is preferably used as GSH F.

The source from which GSH F is derived is not limited and GSH F derived from e.g., microorganisms, animals and plants can be used. GSH F derived from a microorganism is preferable, particularly GSH F derived from a bacterium, and more specifically GSH F derived from at least one selected from the group consisting of *Streptococcus* bacteria, such as *Streptococcus agalactiae, Streptococcus mutans, Streptococcus suis* and *Streptococcus thermophilus; Lactobacillus* bacteria, such as *Lactobacillus plantarum; Desulfotalea* bacteria, such as *Desulfotalea psychrophila; Clostridium* bacteria, such as *Clostridium perfringens; Listeria* bacteria, such as *Listeria innocua* and *Listeria monocytogenes; Enterococcus* bacteria, such as *Enterococcus faecalis* and *Enterococcus faecium; Pasteurella* bacteria, such as *Pasteurella multocida; Mannheimia* bacteria, such as *Mannheimia succiniciprodecens;* and *Haemophilus* bacteria, such as *Haemophilus somnus,* is preferable.

The nucleotide sequence of GSH F derived from *Streptococcus agalactiae* and the amino acid sequence encoded by the nucleotide sequence are specifically represented by SEQ ID NO: 11 and SEQ ID NO: 12, respectively. Furthermore, the nucleotide sequence consisting of 4th base to 2253rd base of SEQ ID NO: 7 is an example of a nucleotide sequence encoding GSH F derived from *Streptococcus agalactiae* consisting of amino acid sequence represented by SEQ ID NO: 12 and being suited in accordance with the use frequency of codons in *Escherichia coli.*

GSH F that can be used includes not only GSH F consisting of the amino acid sequence represented by SEQ ID NO: 12 but also other polypeptides having GSH F activity, such as an active mutant thereof and orthologue from other species. Said other polypeptides having GSH F activity includes a polypeptide exhibiting an activity of preferably 10% or more, preferably 40% or more, more preferably 60% or more, more preferably 80% or more and further preferably 90% or more relative to the activity of the GSH F consisting of the amino acid sequence represented by SEQ ID NO: 12 in the aforementioned activity measurement conditions; and, preferably, a polypeptide exhibiting an activity of preferably 10% or more, preferably 40% or more, more preferably 60% or more, more preferably 80% or more, and further preferably 90% or more relative to the activity to produce oxidized γ-glutamylcysteine from L-cystine and L-glutamic acid in the presence of ATP of the GSH F consisting of the amino acid sequence represented by SEQ ID NO: 12. Examples of said other polypeptides having GSH F activity, such as an active mutant thereof and orthologue from other species, include a polypeptide consisting of an amino acid sequence having addition, deletion or substitution of one or several amino acids in the amino acid sequence represented by SEQ ID NO: 12 (particularly preferably, a polypeptide consisting of an amino acid sequence having substitution, deletion and/or addition, preferably deletion and/or addition of one or several amino acids in total at either one or both of the N terminal and the C terminal of the amino acid sequence represented by SEQ ID NO:12); and a polypeptide consisting of an amino acid sequence having an amino acid identity of 80% or more, preferably 85% or more, more preferably 90% or more, 95% or more, 97% or more, 98% or more or 99% or more with the amino acid sequence represented by SEQ ID NO: 12. Further examples of said other polypeptides that can be used herein include a GSH F-active fragment of at least one polypeptide selected from the group consisting of the polypeptide consisting of the amino acid sequence represented by SEQ ID NO: 12; a polypeptide consisting of an amino acid sequence having addition, deletion or substitution of one or several amino acids in the amino acid sequence represented by SEQ ID NO: 12; and a polypeptide consisting of the amino acid sequence having the aforementioned amino acid identity with the amino acid sequence represented by SEQ ID NO: 12. The fragment may be a polypeptide consisting of amino acids of preferably 400 or more, more preferably 500 or more, and more preferably 600 or more, more preferably 700 or more and more preferably 730 or more. The fragment also has an activity to produce oxidized γ-glutamylcysteine from L-cystine and L-glutamic acid in the presence of ATP. The "amino acid identity" refers to the percentage (%) of the number of identical amino acid residues relative to the total number of amino acid residues of the protein represented by SEQ ID NO: 12 when two amino acid sequences are aligned and arranged so as to obtain a highest degree of matching of amino acids between the two sequences, if necessary by introducing a gap. Furthermore, the substitution of amino acids is made preferably in accordance with conservative amino acid substitution. The preferable range of "several", method for calculating amino acid identity and conservative amino acid substitution mentioned herein are the same as described with respect to GSH I. The aforementioned polypeptides each may be chemically modified.

The nucleotide sequence of a gene (DNA or RNA) encoding the GSH F that can be used for preparation of GSH F is not limited to the nucleotide sequence represented by SEQ ID NO: 11. The nucleotide sequence may be any nucleotide sequence encoding the amino acid sequence of a desired GSH F and being suited to the host organism.

<ADK>

Adenylate kinase (ADK) used in the present invention is an enzyme having an activity to catalyze a reaction for producing one ATP molecule and one AMP molecule from two ADP molecules. ADK may be any enzyme having this activity. The source, structure and other characteristics of ADK are not limited. In the present invention, the activity is referred to as ADK activity. One unit (1 U) of the activity means the activity to produce 1 µmol of AMP at 30°C for one minute and is measured in the following measurement conditions.

### (Measurement condition)

The reaction comprises adding an enzyme solution to a 50 mM tris hydrochloride buffer solution (pH8.0) containing 10 mM ADP and 70 mM magnesium sulfate, and then keeping the reaction solution at 30°C. The reaction is terminated by adding 6N hydrochloric acid. The AMP in the reaction solution is quantified by high-performance liquid chromatography.

The conditions of the high-performance liquid chromatography are as follows. In the conditions, adenosine triphosphate (ATP), adenosine diphosphate (ADP) and adenosine monophosphate (5'-adenyl acid) (AMP) sequentially elute in this order.

### [HPLC conditions]

Column: ODS-HG-3 (4.6mmϕ x 150 mm, manufactured by Nomura Chemical Co., Ltd.);
Eluent: Solution prepared by dissolving potassium dihydrogenphosphate (12.2 g) and sodium heptanesulfonate (3.6 g) with distilled water (1.8 L), controlling the pH of the solution with phosphoric acid to be pH2.8, adding methanol (186 ml) and dissolving it;
Flow rate: 1.0 ml/minute;
Column temperature: 40°C;
Measurement wavelength: 210 nm.

ADK having an ADK activity (specific activity) of 20 U or more per 1 mg of protein is preferably used as ADK.

The source from which ADK is derived is not limited and ADK derived from e.g., microorganisms, animals and plants can be used. ADK derived from a microorganism is preferable, and particularly ADK derived from a bacterium, and more specifically ADK derived from *Eschenichia coli* is preferable.

The nucleotide sequence of ADK derived from *Escherichia coli* and the amino acid sequence encoded by the nucleotide sequence are specifically represented by SEQ ID NO: 13 and SEQ ID NO: 14, respectively.

ADK that can be used includes not only ADK consisting of the amino acid sequence represented by SEQ ID NO: 14 but also other polypeptides having ADK activity, such as an active mutant thereof and orthologue from other species. Said other polypeptides having ADK activity are preferably a polypeptide exhibiting an activity of preferably 10% or more, preferably 40% or more, more preferably 60% or more, more preferably 80% or more and further preferably 90% or more relative to the activity of the ADK consisting of the amino acid sequence represented by SEQ ID NO: 14 in the aforementioned activity measurement conditions. Examples of said other polypeptides having ADK activity, such as an active mutant thereof and orthologue from other species, include a polypeptide consisting of an amino acid sequence having addition, deletion or substitution of one or several amino acids in the amino acid sequence represented by SEQ ID NO: 14 (a polypeptide consisting of an amino acid sequence having substitution, deletion and/or addition, preferably deletion and/or addition of one or several amino acids in total at either one or both of the N terminal and the C terminal of the amino acid sequence represented by SEQ ID NO: 14) and a polypeptide consisting of an amino acid sequence having an amino acid identity of 80% or more, preferably 85% or more, more preferably 90% or more, 95% or more, 97% or more, 98% or more or 99% or more with the amino acid sequence represented by SEQ ID NO: 14. Further examples of said other polypeptides that can be used herein include a ADK-active fragment of at least one polypeptide selected from the group consisting of the polypeptide consisting of the amino acid sequence represented by SEQ ID NO: 14; a polypeptide consisting of an amino acid sequence having addition, deletion or substitution of one or several amino acids in the amino acid sequence represented by SEQ ID NO: 14; and a polypeptide consisting of the amino acid sequence having the aforementioned amino acid identity with the amino acid sequence represented by SEQ ID NO: 14. The fragment may be a polypeptide consisting of amino acids of preferably 100 or more, more preferably 150 or more, more preferably 200 or more. The "amino acid identity" refers to the percentage (%) of the number of identical amino acid residues relative to the total number of amino acid residues of the protein represented by SEQ ID NO: 14, when two amino acid sequences are aligned and arranged so as to obtain a highest degree of matching of amino acids between the two sequences, if necessary by introducing a gap. Furthermore, the substitution of amino acids is made preferably in accordance with conservative amino acid substitution. The preferable range of "several", method for calculating amino acid identity and conservative amino acid substitution mentioned herein are the same as described with respect to GSH I. The aforementioned polypeptides each may be chemically modified.

The nucleotide sequence of a gene (DNA or RNA) encoding the ADK that can be used for preparation of ADK is not limited to the nucleotide sequence represented by SEQ ID NO: 13. The nucleotide sequence may be any nucleotide sequence encoding the amino acid sequence of a desired ADK and being suited to the host organism.

### <PAP>

Polyphosphate-dependent AMP transferase (PAP) used in the present invention is an enzyme having an activity to catalyze a reaction for phosphorylating AMP using a polyphosphoric acid as a phosphoric acid donor to produce ADP. PAP may be any enzyme having this activity. The source, structure and other characteristics of PAP are not limited. In the present invention, the activity is referred to as PAP activity. One unit (1 U) of the activity means the activity to produce 1 µmol of ADP at 30°C for one minute and is measured in the following measurement condition.

### (Measurement condition)

The reaction comprises adding an enzyme solution to a 50 mM tris hydrochloride buffer solution (pH8.0) containing 5 mM sodium metaphosphate, 10 mM AMP and 70 mM magnesium sulfate and then keeping the reaction solution at 30°C. The reaction is terminated by adding 6N hydrochloric acid. ADP in the reaction solution is quantified by high-performance liquid chromatography.

The same conditions for the high-performance liquid chromatography as mentioned in the above activity measurement method for ADK are used.

PAP having a PAP activity (specific activity) of 20 U or more per 1 mg of protein is preferably used as PAP.

The source of PAP is not limited and PAP derived from e.g., microorganisms, animals and plants can be used. PAP derived from a microorganism is preferable, and particularly PAP derived from a bacterium, and more specifically PAP derived from *Acinetobacter johnsonii* is preferable.

The nucleotide sequence of PAP derived from *Acinetobacter johnsonii* and the amino acid sequence encoded by the nucleotide sequence are specifically represented by SEQ ID NO: 15 and SEQ ID NO: 16, respectively. Furthermore, the nucleotide sequence consisting of 4th base to 1428th base of SEQ ID NO: 8 is an example of a nucleotide sequence encoding PAP derived from *Acinetobacter johnsonii* consisting of amino acid sequence represented by SEQ ID NO: 16 and, and being suited in accordance with the use frequency of codons in *Escherichia coli.*

PAP that can be used includes not only PAP consisting of the amino acid sequence represented by SEQ ID NO: 16 but also other polypeptides having PAP activity, such as an active mutant thereof and orthologue from other species. Said other polypeptides having PAP activity include a polypeptide exhibiting an activity of preferably 10% or more, preferably 40% or more, more preferably 60% or more, more preferably 80% or more and further preferably 90% or more relative to the activity of the PAP consisting of the amino acid sequence represented by SEQ ID NO: 16 in the aforementioned activity measurement conditions. Examples of said other polypeptides having PAP activity, such as an active mutant thereof and orthologue from other species, include a polypeptide consisting of an amino acid sequence having addition, deletion or substitution of one or several amino acids in the amino acid sequence represented by SEQ ID NO: 16 (particularly preferably, a polypeptide consisting of an amino acid sequence having substitution, deletion and/or addition, preferably deletion and/or addition of one or several amino acids in total at either one or both of the N terminal and the C terminal of the amino acid sequence represented by SEQ ID NO: 16); and a polypeptide consisting of an amino acid sequence having an amino acid identity of 80% or more, preferably 85% or more, more preferably 90% or more, 95% or more, 97% or more, 98% or more or 99% or more with the amino acid sequence represented by SEQ ID NO: 16. Further examples of said other polypeptides that can be used herein include a PAP-active fragment of at least one polypeptide selected from the group consisting of the polypeptide consisting of the amino acid sequence represented by SEQ ID NO: 16; a polypeptide consisting of an amino acid sequence having addition, deletion or substitution of one or several amino acids in the amino acid sequence represented by SEQ ID NO: 16; and a polypeptide consisting of the amino acid sequence having the aforementioned amino acid identity with the amino acid sequence represented by SEQ ID NO: 16. The fragment may be a polypeptide consisting of amino acids of preferably 250 or more, more preferably 300 or more, more preferably 400 or more and more preferably 450 or more. The "amino acid identity" refers to the percentage (%) of the number of identical amino acid residues relative to the total number of amino acid residues of the protein represented by SEQ ID NO: 16 when two amino acid sequences are aligned and arranged so as to obtain a highest degree of matching of amino acids between the two sequences, if necessary by introducing a gap. Furthermore, the substitution of amino acids is made preferably in accordance with conservative amino acid substitution. The preferable range of "several", method for calculating amino acid identity and conservative amino acid substitution mentioned herein are the same as described with respect to GSH I. The aforementioned polypeptides each may be appropriately chemically modified.

The nucleotide sequence of a gene (DNA or RNA) encoding the PAP that can be used for preparation of PAP is not limited to the nucleotide sequence represented by SEQ ID NO: 15. The nucleotide sequence may be any nucleotide sequence encoding the amino acid sequence of a desired PAP and being suited to the host organism.

### <Preparation of enzymes>

The methods for obtaining the aforementioned individual enzymes used in the present invention are not limited. Each of the enzymes can be prepared from organisms having the activity of the enzyme, for example, a wild strain or variant strain of a microorganism. As the organism having the activity of a desired enzyme, either an organism that natively has the enzymatic activity or an organism that has been augmented in the enzymatic activity may be used. Examples of the organism that has been augmented in the enzymatic activity include recombinant biological cells, in which the expression of a gene encoding each of the enzymes has been augmented by genetic engineering technique. Note that "the organism that has been augmented in the enzymatic activity" include not only an organism that natively has the enzymatic activity, in which the enzymatic activity has been augmented further, but also an organism that natively lacks in the enzymatic activity, in which the enzymatic activity has been added.

The recombinant biological cell obtained by genetic engineering technique typically refers to a recombinant biological cell having an ability to produce a desired enzyme. Such a cell can be obtained by inserting a gene (DNA or RNA) encoding the desired enzyme into an appropriate vector to prepare a recombinant vector and transforming an appropriate host cell with the recombinant vector. The desired enzyme can be produced by culturing the recombinant biological cell. Examples of the host cell include bacterial cells, yeast cells, filamentous fungus cells, plant cells and animal cells. In view of introduction and expression efficiency, bacterial cells are preferable, and particularly *Escherichia coli* cell is preferable.

### <Step A and step A'>

A method for producing oxidized γ-glutamylcysteine according to the present invention is characterized by comprising step A of producing oxidized γ-glutamylcysteine by reacting L-cystine and L-glutamic acid in the presence of at least one enzyme selected from the group consisting of γ-glutamylcysteine synthetase and bifunctional glutathione synthetase, and ATP.

The method for producing oxidized γ-glutamylcysteine according to the present invention is also characterized by comprising step A' of producing oxidized γ-glutamylcysteine by reacting L-cystine and L-glutamic acid. Step A' may be a step based on an enzymatic reaction or a step based on a chemical reaction using no enzyme; preferably a step based on an enzymatic reaction and particularly preferably the step A as mentioned above. The enzymatic reaction is advantageous over the chemical synthesis reaction, for the reason that e.g., protection of a substrate compound with a functional group is not required, or specificity of a reaction is high.

Examples of step A' based on a chemical reaction using no enzyme include, but not particularly limited to, a step comprising reacting L-cystine having two carboxyl groups protected with an appropriate protecting group with L-glutamic acid having an α-carboxyl group and an amino group protected with appropriate protecting groups and subjecting each of the two amino groups of one L-cystine molecule and a γ-carboxyl group of one L-glutamic acid molecule to dehydration condensation to form peptide bonds. In the step, if necessary, one or more protecting groups are removed after the dehydration condensation reaction. The protecting group for a carboxyl group may be a commonly known protecting group for a carboxyl group, such as a benzyl group. The protecting group for an amino group may be a commonly known protecting group for an amino group, such as t-butoxycarbonyl (Boc) group and a 9-fluorenylmethoxycarbonyl (Fmoc) group.

Step A may use, as GSH I and/or GSH F, a cell of an organism having GSH I and/or GSH F activity, which may be a living cell or may be a dead but undamaged cell. Alternatively, the step may use GSH I and/or GSH F present outside the cell, more specifically, a ground material (i.e., crushed material) of the cell of the organism. Alternatively, the step may use GSH I and/or GSH F protein, which is isolated from the cell and purified. Herein, the purification degree of a protein having GSH I and/or GSH F activity is not limited, and the step may use GSH I and/or GSH F roughly purified. Step A preferably uses no living cell having GSH I and/or GSH F activity, and more preferably, uses neither a living cell nor an undamaged dead cell having GSH I and/or GSH F activity. In particular, step A preferably uses the extracellular GSH I and/or GSH F, more specifically, GSH I and/or GSH F in a cell ground material or GSH I and/or GSH F protein which has been isolated from a cell and purified, because in this case, in comparison with the case of using a living cell, it is considered that starting L-cystine and the reaction product, oxidized γ-glutamylcysteine, are easily maintained in an oxidized state in the reaction mixture and thus oxidized γ-glutamylcysteine can be efficiently obtained. In the case where step A uses extracellular GSH I and/or GSH F, in which a reduction action due to a living cell is not present in the reaction system, the above action presumably takes place. This tendency is particularly remarkable when the reaction mixture is placed in the conditions where the reaction mixture is allowed to be in contact with air. Furthermore, in the case where step A uses a living cell having GSH I and/or GSH F activity, adenosine monophosphate (AMP) tends to be easily decomposed in the reaction system. If AMP, which is one of the intermediates of the ATP regeneration reaction (described later), is decomposed, the efficiency of ATP regeneration reaction would decrease. In contrast, in the case where step A uses extracellular GSH I and/or GSH F, AMP is rarely decomposed and the ATP regeneration reaction can efficiently proceed. Because of this, use of extracellular GSH I and/or GSH F is preferable. More specifically, when step A of the present invention is carried out by using extracellular GSH I and/or GSH F, decomposition of AMP and reduction of oxidized γ-glutamylcysteine can be simultaneously suppressed.

In the specification, "ground" (i.e., "crushed") of a cell refers to a treatment by which the surface structure of a cell is damaged to the extent that an enzyme produced within the cell is accessible from outside the cell, and the cell is not necessarily fragmented. In the specification, "ground material" ("crushed material") of a cell refers to a ground cell resulting from the grinding treatment. Grinding (crushing) treatment of a cell can be carried out by applying a single grinding treatment or a plurality of grinding treatments in an appropriate order. Examples of the grinding treatment of a cell include a physical treatment, a chemical treatment and an enzymatic treatment. Examples of the physical treatment include treatments using a high-pressure homogenizer, an ultrasonic homogenizer, a French press, a ball mill and a combination of these treatments. Examples of the chemical treatment include a treatment using an acid (preferably a strong acid), such as hydrochloric acid and sulfuric acid, a treatment using a base (preferably a strong base), such as sodium hydroxide and potassium hydroxide, and a combination of these treatments. Examples of the enzymatic treatment include treatments using, lysozyme, zymolyase, glucanase, protease and cellulase, and a combination of these treatments.

L-cystine and L-glutamic acid used as a substrate in step A and/or step A' and ATP used in step A each can be added in the reaction system in various forms, such as a salt, free form and a solvate (e.g., a hydrate).

The reaction system (for example, reaction mixture) in step A and/or step A' does not substantially contain L-cysteine. More specifically, in the reaction system of step A and/or step A', L-cystine is contained in a percentage of 70 mol% or more relative to the total molar amount of L-cystine and L-cysteine, more preferably 80 mol% or more, more preferably 90 mol% or more, further preferably 95 mol% or more, further preferably 98 mol% or more and most preferably 100 mol%. The requirement that the percentage of L-cystine relative to the total molar amount of L-cystine and L-cysteine falls within the above range is satisfied preferably at least at the starting time of the reaction in step A and/or step A' and more preferably during the period from the starting time to completion of the reaction in step A and/or step A'.

In step A, GSH I and/or GSH F is reacted with a reaction system containing L-cystine and L-glutamic acid in the presence of ATP. The reaction can be carried out in a reaction mixture containing a solvent, such as water, controlled at appropriate pH. The conditions at this time are not limited; however a substrate concentration (total concentration of L-cystine and L-glutamic acid) can be set to be preferably about 0.1 to 99 wt% and more preferably 1 to 20 wt%. At the starting time of the reaction, the quantitative ratio (molar ratio) of L-cystine and L-glutamic acid in the substrate can be L-cystine: L-glutamic acid = 1: about 2, for example, L-cystine: L-glutamic acid = 1: 1 to 4, and preferably 1: 1.5 to 3. The reaction temperature can be preferably 10 to 60°C and more preferably 20 to 50°C. The pH of the reaction is preferably 4 to 11 and more preferably 6 to 9. The reaction time can be preferably 1 to 120 hours and more preferably 1 to 72 hours. The reaction in step A and/or step A' is preferably carried out while allowing the reaction mixture in contact with air, because, under this condition, starting L-cystine and the reaction product, oxidized γ-glutamylcysteine, are easily maintained in an oxidized state. After completion of the reaction in step A and/or step A', reduced γ-glutamylcysteine is not substantially contained in the reaction system (for example, reaction mixture), more specifically, oxidized γ-glutamylcysteine is contained in a percentage of 70 mol% or more relative to the total molar amount of oxidized γ-glutamylcysteine and reduced γ-glutamylcysteine in the reaction system after completion of the reaction in step A and/or step A', more preferably 80 mol% or more, more preferably 90 mol% or more, further preferably 95 mol% or more, further preferably 98 mol% or more, and most preferably 100 mol%.

The concentration of each of the enzymes in the reaction mixture can be appropriately controlled. For example, the lower limit thereof in terms of protein is 1 µg/ml or more and the upper limit is not specified; however, it can be appropriately controlled in the range up to preferably 100 mg/ml or less. When GSH I is used in step A, the GSH I activity in the reaction mixture in step A is not limited; however, the lower limit thereof is preferably 0.1 U/ml or more and the upper limit (not particularly specified) can be usually 10000 U/ml or less. When GSH F is used in step A, GSH F activity in the reaction mixture in step A is not limited; however, the lower limit thereof is preferably 0.1 U/ml or more and the upper limit (not particularly specified) can be set at usually 10000 U/ml or less.

The concentration of ATP in the reaction mixture can be appropriately controlled depending upon the concentration of cystine serving as a substrate and the presence or absence of the ATP regenerating system. In step A, ATP is consumed twice by molar ratio compared to cystine. When step A is carried out in conjugation with the ATP regeneration reaction, the addition amount of ATP relative to cystine can be greatly reduced. Then, the upper limit of the ATP concentration in the reaction mixture in step A, which is not limited, is preferably 4 fold or less and more preferably 2.2 fold or less as large as the cystine molar concentration. The lower limit of the ATP concentration in the reaction mixture in step A, which is not limited, is preferably 0.0001 fold or more, more preferably 0.001 fold or more and further preferably 0.01 fold or more as large as the cystine molar concentration.

### <Step B and step B'>

A method for producing oxidized glutathione (GSSG) according to the present invention is characterized by comprising step B of reacting oxidized γ-glutamylcysteine and glycine in the presence of glutathione synthetase (GSH II) and ATP to produce oxidized glutathione.

A method for producing GSSG according to the present invention is also characterized by comprising step B' of reacting oxidized γ-glutamylcysteine and glycine in the presence of GSH II and ATP to produce oxidized glutathione. Step B' may be a step based on an enzymatic reaction or a step based on a chemical reaction using no enzyme. Step B' is preferably a step based on an enzymatic reaction and is particularly preferably the step B mentioned above. The enzymatic reaction is advantageous over the chemical synthesis reaction, for the reason that e.g., protection of a substrate compound with a functional group is not required, or specificity of a reaction is high.

Examples of step B' based on a chemical reaction using no enzyme include, but not particularly limited to, a step comprising reacting oxidized γ-glutamylcysteine, in which an α-carboxyl group and an amino group in an L-glutamic acid residue are protected with appropriate protecting groups, and glycine, in which a carboxyl group is protected with an appropriate protecting group, and subjecting each of the two carboxyl groups in one oxidized γ-glutamylcysteine molecule and an amino group of one glycine molecule to dehydration condensation to form a peptide bond. In the step, if necessary, one or more protecting groups are removed after the dehydration condensation reaction. The protecting group for a carboxyl group may be a commonly known protecting group for a carboxyl group, such as a benzyl group. The protecting group for an amino group may be a commonly known protecting group for an amino group, such as t-butoxycarbonyl (Boc) group and a 9-fluorenylmethoxycarbonyl (Fmoc) group.

Step B may use, as GSH II, a cell of an organism having GSH II activity, which may be a living cell or may be a dead but undamaged cell. Alternatively, the step may use GSH II present outside the cell, more specifically, a ground material (i.e., crushed material) of the cell of the organism. Alternatively, the step may use GSH II protein, which is isolated from the cell and purified. Herein, the purification degree of a protein having GSH II activity is not limited, and the step may use GSH II roughly purified. Herein, the "ground material" of a cell is the same as defined above. Step B preferably uses no living cell having GSH II activity, and more preferably, uses neither a living cell nor an undamaged dead cell having GSH II activity. In particular, step B preferably uses the extracellular GSH II, more specifically, GSH II in a cell ground material or GSH II protein which has been isolated from a cell and purified, because in this case in comparison with the case of using a living cell, it is considered that starting oxidized γ-glutamylcysteine and the reaction product, oxidized glutathione, are easily maintained in an oxidized state in the reaction mixture and thus oxidized glutathione can be efficiently obtained. In the case where step B uses the extracellular GSH II, in which a reduction action due to a living cell is not present in the reaction system, the above action presumably takes place. This tendency is particularly remarkable when the reaction mixture is placed in the conditions where the reaction mixture is allowed to be in contact with air. Furthermore, in the case where step B uses a living cell having GSH II activity, adenosine monophosphate (AMP) tends to be easily decomposed in the reaction system. If AMP is decomposed, the efficiency of ATP regeneration reaction would decrease. In contrast, in the case where step B uses extracellular GSH II, AMP is rarely decomposed and the ATP regeneration reaction can efficiently proceed. Because of this, use of extracellular GSH II is preferable. More specifically, when step B of the present invention is carried out by using extracellular GSH II, decomposition of AMP and reduction of oxidized glutathione and oxidized γ-glutamylcysteine can be simultaneously suppressed.

Oxidized γ-glutamylcysteine and glycine used as substrates in Step B and/or step B' and ATP used in step B can be added in a reaction system in various forms, such as a salt, free form and a solvate (e.g., a hydrate).

The reaction system (for example, the reaction mixture) in step B and/or step B' does not substantially contain reduced γ-glutamylcysteine. More specifically, in the reaction system of step B and/or step B', oxidized γ-glutamylcysteine is contained in a percentage of 70 mol% or more relative to the total molar amount of oxidized γ-glutamylcysteine and reduced γ-glutamylcysteine, more preferably 80 mol% or more, more preferably 90 mol% or more, further preferably 95 mol% or more, further preferably 98 mol% or more and most preferably 100 mol%. The requirement that the percentage of oxidized γ-glutamylcysteine relative to the total molar amount of oxidized γ-glutamylcysteine and reduced γ-glutamylcysteine falls within the above range is satisfied preferably at least at the starting time of the reaction in step B and/or step B' and more preferably during the period from the starting time to completion of the reaction in step B and/or step B'.

In step B, GSH II is reacted with a reaction system containing oxidized γ-glutamylcysteine and glycine in the presence of ATP. The reaction can be carried out in a reaction mixture containing a solvent, such as water, controlled at appropriate pH. The conditions at this time are not limited; however, a substrate concentration (total concentration of γ-glutamylcysteine and glycine) can be set to be preferably about 0.1 to 99 wt% and more preferably 1 to 20 wt%. At the starting time of the reaction, the quantitative ratio (molar ratio) of oxidized γ-glutamylcysteine and glycine in the substrate can be oxidized γ-glutamylcysteine: glycine = 1: about 2, for example, oxidized γ-glutamylcysteine: glycine = 1: 1 to 4, and preferably 1: 1.5 to 3. The reaction temperature can be preferably 10 to 60°C and more preferably 20 to 50°C. The pH of the reaction is preferably 4 to 11 and more preferably 6 to 9. The reaction time can be preferably 1 to 120 hours and more preferably 1 to 72 hours. The reaction in step B and/or step B' is preferably carried out while allowing the reaction mixture in contact with air, because, under this condition, starting oxidized γ-glutamylcysteine and the reaction product, oxidized glutathione, are easily maintained in an oxidized state. After completion of the reaction in step B and/or step B', reduced glutathione is not substantially contained in the reaction system (for example, the reaction mixture), more specifically, oxidized glutathione is contained in a percentage of 70 mol% or more relative to the total molar amount of oxidized glutathione and reduced glutathione, in the reaction system after completion of the reaction in step B and/or step B', more preferably 80 mol% or more, more preferably 90 mol% or more, further preferably 95 mol% or more, further preferably 98 mol% or more, and most preferably 100 mol%.

Oxidized γ-glutamylcysteine serving as a starting material in step B can be obtained in step A. Oxidized γ-glutamylcysteine may be separated from the reaction mixture after completion of step A and then used in step B. After completion of step A, step B may be carried out without separating oxidized γ-glutamylcysteine by adding GSH II and glycine to the reaction mixture of the step A. Alternatively, step A is carried out in the conditions deficient in at least one of GSH II and glycine, in other words, in the conditions in which step B cannot proceed; and then step B may be started by supplementing the deficient element in the above reaction, to the reaction mixture without separating oxidized γ-glutamylcysteine from the reaction mixture after completion of step A.

The reactions of step A and step B are not necessarily carried out in order and may be simultaneously carried out. More specifically, a starting mixture containing L-cystine, L-glutamic acid and glycine may be reacted in the presence of the enzymes used in step A and the enzyme used in step B and ATP. This embodiment is also one of the embodiments of the present invention in which oxidized γ-glutamylcysteine used as a starting material in step B, is produced by step A.

Similarly, oxidized γ-glutamylcysteine serving as a starting material in step B' can be obtained in step A'. Oxidized γ-glutamylcysteine may be separated from the reaction mixture after completion of step A' and then used in step B'. After completion of step A', step B' may be carried out without separating oxidized γ-glutamylcysteine by adding glycine to the reaction mixture of step A' and appropriately controlling reaction conditions. The reactions of step A' and step B' are not necessarily carried out in order and may be simultaneously carried out. More specifically, a starting mixture containing L-cystine, L-glutamic acid and glycine may be reacted. This embodiment is also one of the embodiments of the present invention in which oxidized γ-glutamylcysteine serving as a starting material in step B' is produced by step A'.

The concentration of each of the enzymes in a reaction mixture can be appropriately controlled. For example, the lower limit thereof in terms of protein is 1 µg/ml or more and the upper limit is not particularly specified; however, it can be appropriately controlled in the range up to preferably 100 mg/ml or less. When step B uses GSH II, the GSH II activity in the reaction mixture in step B is not limited; however, the lower limit thereof is preferably 0.1 U/ml or more and the upper limit (not particularly specified) can be set at usually 10000 U/ml or less.

The concentration of ATP in the reaction mixture can be appropriately controlled depending upon the concentration of oxidized γ-glutamylcysteine serving as a substrate and the presence or absence of the ATP regenerating system. In step B, ATP is consumed twice by molar ratio compared to oxidized γ-glutamylcysteine. When step B is carried out in conjugation with the ATP regeneration reaction, the addition amount of ATP relative to oxidized γ-glutamylcysteine can be greatly reduced. Then, the upper limit of the ATP concentration in the reaction mixture in step B, which is not limited, is preferably 4 fold or less and more preferably 2.2 fold or less as large as the molar concentration of oxidized γ-glutamylcysteine. The lower limit of concentration of ATP in the reaction mixture in step B, which is not limited, is preferably 0.0001 fold or more, more preferably 0.001 fold or more and further preferably 0.01 fold or more as large as the molar concentration of oxidized γ-glutamylcysteine.

### <ATP regeneration reaction>

Steps A and B each are a step in which ATP is consumed to produce ADP. Since ATP is a relatively expensive material, steps A and B are preferably carried out in conjugation with an ATP regeneration reaction for regenerating ATP from ADP produced in the steps.

The ATP regeneration reaction includes a reaction for regenerating ATP from ADP using a phosphate group supply source and phosphotransferase.

A scheme of ATP regeneration reaction is shown below. The scheme shows an ATP regeneration reaction using a polyphosphoric acid (condensed phosphoric acid) as a phosphate group supply source and a combination of polyphosphate-dependent AMP transferase (PAP) and adenylate kinase (ADK) as a phosphotransferase.

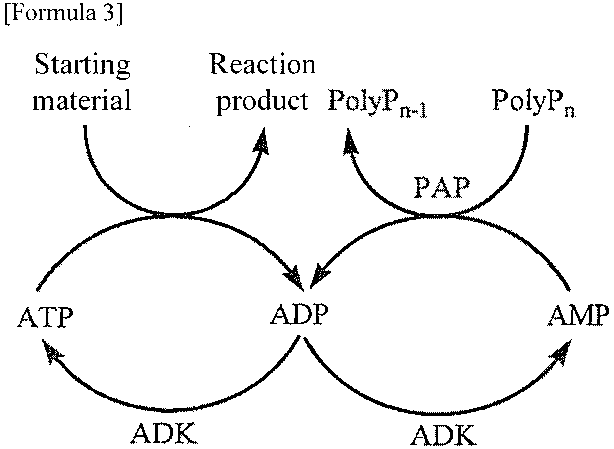

In the scheme, AMP represents adenosine monophosphate, PAP a polyphosphate-dependent AMP transferase, ADK an adenylate kinase, PolyPₙ a polyphosphoric acid (condensed phosphoric acid) in which "n" represents the number of phosphorus atoms, PolyPₙ₋₁ a polyphosphoric acid (condensed phosphoric acid) in which "n-1" represents the number of phosphorus atoms, "starting material" a starting material of step A or B and "reaction product" a reaction product in step A or B.

More specifically, when step A and B are carried out in the presence of a polyphosphoric acid, PAP and ADK, ATP is consumed to produce ADP; ADP is then converted into ATP and AMP by the action of ADK; and AMP produced by the action of ADK is converted into ADP by the action of PAP. This ATP regeneration reaction can be carried out in conjugation with the reactions of steps A and B.

This reaction may use, as ADK and PAP, a cell of an organism having the activities of these enzymes, which may be living or may be dead but undamaged. Alternatively, the reaction may use ADK and/or PAP present outside the cell, more specifically, a ground material (i.e., crushed material) of the cell. Alternatively, the reaction may use ADK and/or PAP protein, which is isolated from the cell and purified. Herein, the degree of purification of a protein having ADK or PAP activity is not limited, and the reaction may use ADK or PAP roughly purified. The "ground material" of a cell is the same as defined above. Step A or B preferably uses no living cell having ADK and/or PAP activity, and more preferably, uses neither a living cell nor an undamaged dead cell having ADK and/or PAP activity.

The ATP regeneration reaction preferably uses, as the enzyme, extracellular ADK and/or PAP, more specifically, ADK and/or PAP present in a cell ground material or ADK and/or PAP protein isolated from the cell. When the ATP regeneration reaction uses a living cell having ADK and/or PAP activity, AMP tends to be easily decomposed. When AMP is decomposed, the efficiency of ATP regeneration reaction would decrease. In contrast, in the case where the ATP regeneration reaction uses extracellular ADK and/or PAP, AMP is rarely decomposed and the ATP regeneration reaction can efficiently proceed. Because of this, use of extracellular ADK and/or PAP is preferable. Note that, the case where step A and/or step B is carried out without using a living cell is advantageous since a reduction action due to a living cell is not present and thus reduction of oxidized γ-glutamylcysteine and/or oxidized glutathione is suppressed. More specifically, when step A and/or step B of the present invention is carried out while using extracellular ADK and/or PAP in the ATP regeneration reaction, decomposition of AMP and reduction of oxidized γ-glutamylcysteine and/or oxidized glutathione can be simultaneously suppressed.

The concentration of each of the enzymes in the reaction mixture used in the ATP regeneration reaction can be appropriately controlled. For example, the lower limit thereof in terms of protein is 1 µg/ml or more and the upper limit is not particularly specified; however, it can be appropriately controlled in the range up to preferably 100 mg/m or less. When step A or B is carried out in conjugation with the ATP regeneration reaction, the ADK activity in the reaction mixture is not limited; however, the lower limit thereof is preferably 5 U/ml or more. The upper limit is not particularly specified; however, it can be usually set to be 500000 U/ml or less. The PAP activity in the reaction mixture is not limited; however, the lower limit thereof is preferably 1 U/ml or more. The upper limit is not particularly specified, however, the upper limit can be usually set to be 100000 U/ml or less.

The addition amount of polyphosphoric acid (condensed phosphoric acid) may be appropriately controlled in accordance with the amount of substrate in the reaction. The polyphosphoric acid can be added in various forms, such as a salt (e.g., sodium salt, potassium salt), free form and a solvate (e.g., a hydrate). The degree of polymerization (the number of phosphorus atoms per molecule) of the polyphosphoric acid is not limited. Sodium metaphosphate used in Examples and Comparative Examples was a mixture of various condensed sodium phosphates different in degree of polymerization.

### Examples

### <Experiment 1>

### Preparation of γ-glutamylcysteine synthetase (GSH I) derived from Escherichia coli K12 strain

A DNA primer (Primer-1: SEQ ID NO: 2) having a restriction enzyme SacI cleavage site and an SD sequence bound to the nucleotide sequence corresponding to the N terminal portion of the GSH I gene (SEQ ID NO: 1); and a DNA primer (Primer-2: SEQ ID NO: 3) having a restriction enzyme KpnI cleavage site bound to the nucleotide sequence corresponding to the C terminal portion of the GSH I gene (SEQ ID NO: 1) were prepared. Using these DNA primers, DNA between these sequences was amplified by PCR to obtain a DNA fragment containing a full length of the GSH I gene. The template used in the PCR amplification was the genomic DNA of *Escherichia coli* K12 strain. The result of analysis of the nucleotide sequence of the obtained DNA fragment showed that the full length of GSH I gene (SEQ ID NO: 1) was contained. The obtained DNA fragment was inserted between a SacI recognition site and a KpnI recognition site present at the downstream of a lac promoter of plasmid pUC18 (GenBank Accession No. L09136, manufactured by Takara Bio Inc.) to construct a recombinant vector, pUCGSHI. *E. coli* HB101 competent cell (manufactured by Takara Bio Inc.) was transformed with the recombinant vector pUCGSHI to obtain *E. coli* HB101 (pUCGSHI). The obtained transformant was inoculated on 50 ml of 2 x YT culture medium (tryptone 1.6%, yeast extract 1.0%, NaCl 0.5%, pH7.0) containing 200 µg/ml ampicillin and subjected to shaking culture at 37°C for 24 hours. Then, enzyme activity was measured. As a result, GSH I activity was 5 U/ml and the activity of ADK derived from *Escherichia coli* used as a host cell was 90 U/ml. Subsequently, bacterial cells were centrifugally collected, suspended in 2.5 ml of a 100 mM phosphate buffer (pH7.0) and sonicated to obtain an enzyme solution.

### <Experiment 2>

### Preparation of glutathione synthetase (GSH II) derived from Escherichia coli K12 strain

A DNA primer (Primer-3: SEQ ID NO: 5) having a restriction enzyme NdeI cleavage site bound to the nucleotide sequence corresponding to the N terminal portion of a GSH II gene (SEQ ID NO: 4); and a DNA primer (Primer-4: SEQ ID NO: 6) having a restriction enzyme EcoRI cleavage site bound to the nucleotide sequence corresponding to the C terminal portion of GSH II gene (SEQ ID NO: 4) were prepared. Using these DNA primers, DNA between these sequences was amplified by PCR to obtain a DNA fragment containing a full length of the GSH II gene. The template used in the PCR amplification was the genomic DNA of *Escherichia coli* K12 strain. The result of analysis of the nucleotide sequence of the obtained DNA fragment showed that the full length of GSH II gene (SEQ ID NO: 4) was contained. The obtained DNA fragment was inserted between a NdeI recognition site and an EcoRI recognition site present at the downstream of a lac promoter of plasmid pUCN18 to construct a recombinant vector, pNGSHII. The plasmid pUCN18 was a plasmid obtained by substituting "T" at the 185th positions of pUC18 (GenBank Accession No. L09136 manufactured by Takara Bio Inc.) with "A" to destruct an NdeI site and further substituting "GC" at the 471-472nd positions with "TG" to newly introduce a NdeI site. *E. coli* HB101 competent cell (manufactured by Takara Bio Inc.) was transformed with the recombinant vector pNGSHII to obtain *E. coli* HB101 (pNGSHII). The obtained transformant was inoculated on 50 ml of 2 x YT culture medium (tryptone 1.6%, yeast extract 1.0%, NaCl 0.5%, pH7.0) containing 200 µg/ml ampicillin and subjected to shaking culture at 37°C for 24 hours. Then, enzyme activity was measured. As a result, GSH II activity was 5 U/ml and the activity of ADK derived from *Escherichia coli* used as a host cell was 90 U/ml. Subsequently, bacterial cells were centrifugally collected, suspended in 2.5 ml of a 100 mM phosphate buffer (pH7.0) and sonicated to obtain an enzyme solution.

### <Experiment 3>

### Preparation of bifunctional glutathione synthetase (GSH F) derived from Streptococcus agalactiae

A GSH F gene fragment (SEQ ID NO: 7) derived from *Streptococcus agalactiae* was manufactured in accordance with a gene synthesis method (by Eurogentec). In this GSH F gene fragment, the codons were optimized for expression in *Escherichia coli;* and a restriction enzyme NdeI cleavage site was bound to the nucleotide sequence corresponding to the N terminal portion; and a restriction enzyme EcoRI cleavage site was bound to the nucleotide sequence corresponding to the C terminal portion. The obtained DNA fragment was inserted between a NdeI recognition site and an EcoRI recognition site present at the downstream of a lac promoter of plasmid pUCN18 to construct a recombinant vector, pNGSHF. The plasmid pUCN18 was a plasmid obtained by substituting "T" at the 185th position of pUC18 (GenBank Accession No. L09136 manufactured by Takara Bio Inc.) with "A" to destruct an NdeI site and further substituting "GC" at the 471-472nd positions with "TG" to newly introduce a NdeI site. *E. coli* HB101 competent cell (manufactured by Takara Bio Inc.) was transformed with the recombinant vector pNGSHF to obtain *E. coli* HB101 (pNGSHF). The obtained transformant was inoculated on 50 ml of 2 x YT culture medium (tryptone 1.6%, yeast extract 1.0%, NaCl 0.5%, pH7.0) containing 200 µg/ml ampicillin and subjected to shaking culture at 37°C for 24 hours. Then, enzyme activity was measured. As a result, GSH F activity was 3 U/ml and the activity of ADK derived from *Escherichia coli* used as a host cell was 90 U/ml. Subsequently, bacterial cells were centrifugally collected, suspended in 2.5 ml of a 100 mM phosphate buffer (pH7.0) and sonicated to obtain an enzyme solution.

### <Experiment 4>

### Preparation of AMP phosphotransferase (PAP) derived from Acinetobacter johnsonii

A PAP gene fragment (SEQ ID NO: 8) was manufactured in accordance with a gene synthesis method (by Eurogentec). In this PAP gene fragment, the codons were optimized for expression in *Escherichia coli;* and a restriction enzyme NdeI cleavage site was bound to the nucleotide sequence corresponding to the N terminal portion; and a restriction enzyme EcoRI cleavage site was bound to the nucleotide sequence corresponding to the C terminal portion. The obtained DNA fragment was inserted between a NdeI recognition site and an EcoRI recognition site present at the downstream of a lac promoter of plasmid pUCN18 to construct a recombinant vector, pNPAP. The plasmid pUCN18 was a plasmid obtained by substituting "T" at the 185th position of pUC18 (GenBank Accession No. L09136 manufactured by Takara Bio Inc.) with "A" to destruct an NdeI site and further substituting "GC" at the 471-472nd positions with "TG" to newly introduce a NdeI site. *E. coli* HB101 competent cell (manufactured by Takara Bio Inc.) was transformed with the recombinant vector pNPAP to obtain *E. coli* HB101 (pNPAP). The obtained transformant was inoculated on 50 ml of 2 x YT culture medium (tryptone 1.6%, yeast extract 1.0%, NaCl 0.5%, pH7.0) containing, 200 µg/ml ampicillin and subjected to shaking culture at 37°C for 24 hours. Then, enzyme activity was measured. As a result, PAP activity was 40 U/ml and the activity of ADK derived from *Escherichia coli* used as a host cell was 90 U/ml. Subsequently, bacterial cells were centrifugally collected, suspended in 2.5 ml of a 100 mM phosphate buffer (pH7.0) and sonicated to obtain an enzyme solution.

### <Calculation of yield>

A method of calculating the yield of each of the compounds obtained in the Experiments herein was as follows.

A reaction product was quantified based on analysis by high-performance liquid chromatography and then the yield thereof was obtained by the following expression:
Yield: the production amount (mol) of compound/starting L-cystine (mol) x 100

The conditions of the above high-performance liquid chromatography are as follows. In the elution conditions, reduced glutathione (GSH), reduced γ-glutamylcysteine (γ-GC), oxidized γ-GC, oxidized glutathione (GSSG) sequentially elute in this order.

### [Analysis of yield]

Column: ODS-HG-3 (4.6mmϕ x 150 mm, manufactured by Nomura Chemical Co., Ltd.);
Eluent: Solution prepared by dissolving potassium dihydrogenphosphate (12.2 g) and sodium heptane sulfonate (3.6 g) in distilled water (1.8 L), controlling pH of the solution to be pH 2.8 with phosphoric acid and further adding methanol (186 ml);
Flow rate: 1.0 ml/minute;
Column temperature: 40°C;
Measurement wavelength: 210 nm.

### <Example 1>

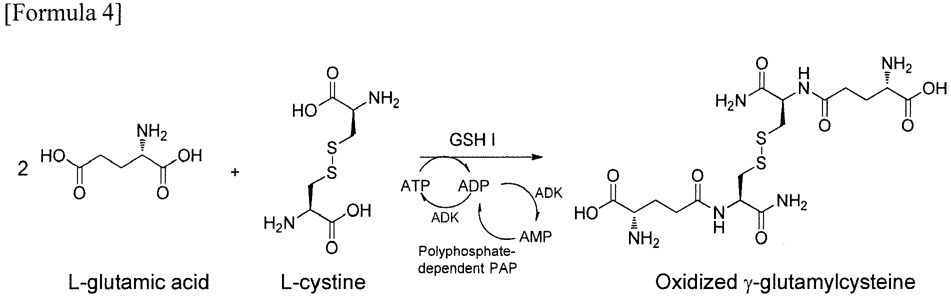

Monosodium L-glutamate monohydrate (0.3629 g (2.15 mmol)), L-cystine dihydrochloride (0.3113 g (0.99 mmol)), magnesium sulfate hepta hydrate (0.7079 g), ATP (0.0583 g (0.11 mmol)), sodium metaphosphate (0.8 g) and distilled water (12 g) were mixed. The pH of the mixture was controlled to be 7.5 with a 15 wt% aqueous sodium hydroxide solution (0.8 g). To the mixture, the γ-glutamylcysteine synthetase (GSH I) solution (2 g) prepared in Experiment 1 and the PAP solution (2 g) prepared in Experiment 4 were added and a reaction was initiated at a reaction temperature of 30°C. The reaction was carried out for one hour and then the reaction solution was analyzed. As a result, production of oxidized γ-glutamylcysteine was verified. The yield after a one-hour reaction based on the starting L-cystine was 20 mol% and the yield after a 22-hour reaction was 95 mol%. In this case, the yield of reduced γ-glutamylcysteine after the 22-hour reaction based on the starting L-cystine was 1 mol% or less.

Since the GSH I solution prepared in Experiment 1 and the PAP solution prepared in Experiment 4 contained ADK derived from *Escherichia coli* used as a host cell, it was not necessary to separately prepare ADK.

### <Example 2>

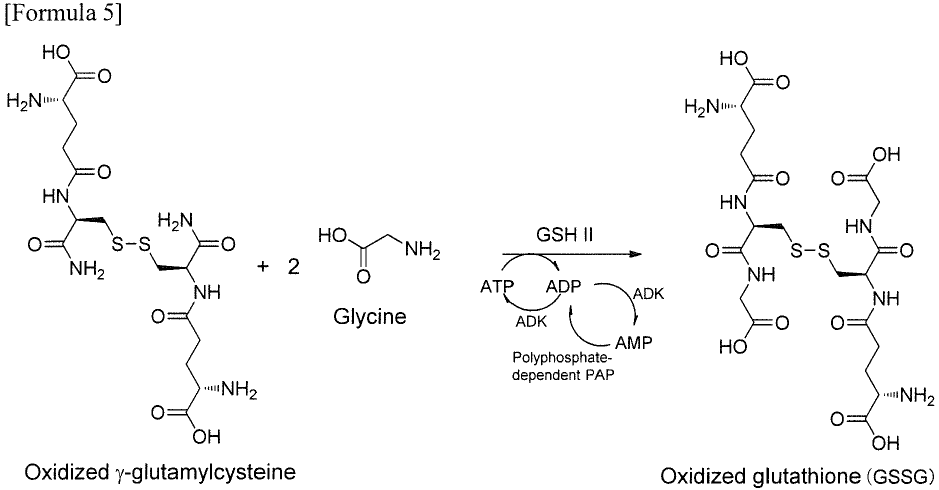

After the 22-hour reaction in Example 1, to the reaction solution, glycine (0.19 g (2.53 mmol)), the glutathione synthetase (GSH II) solution (2 g) prepared in Experiment 2 and the PAP solution (2 g) prepared in Experiment 4 were added and a reaction was initiated. In this case, the pH of the mixture was controlled to be 7.5 with a 15 wt% aqueous sodium hydroxide solution (0.2 g). The reaction was carried out for one hour and then the reaction solution was analyzed. As a result, production of oxidized glutathione was verified. The yield after a one-hour reaction based on the starting L-cystine was 20 mol% and the yield after a 6-hour reaction was 86 mol%. In this case, the yield of reduced glutathione after the 6-hour reaction based on the starting L-cystine was 1 mol% or less.

Since ADK derived from *Escherichia coli* used as a host cell was contained in the GSH II solution prepared in Experiment 2 and the PAP solution prepared in Experiment 4, it was not necessary to separately prepare ADK.

### <Example 3>

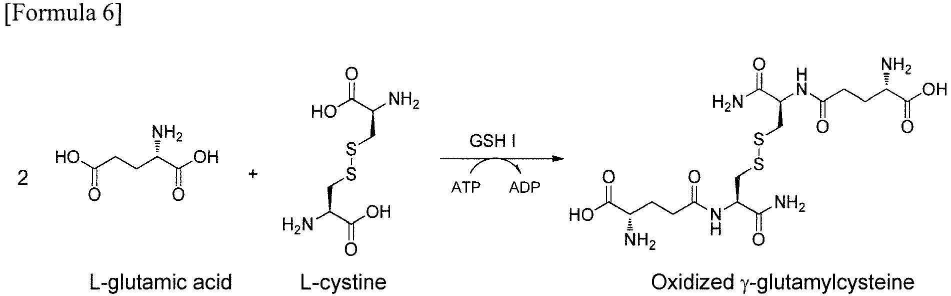

Monosodium L-glutamate monohydrate (0.0717 g (0.42 mmol)), L-cystine dihydrochloride (0.0643 g (0.21 mmol)), magnesium sulfate hepta hydrate (0.2053 g), ATP (0.24 g (0.44 mmol)), and distilled water (17 g) were mixed. The pH of the mixture was controlled to be 7.5 with a 15 wt% aqueous sodium hydroxide solution (0.26 g). To the mixture, the γ-glutamylcysteine synthetase (GSH I) solution (2 g) prepared in Experiment 1 was added and a reaction was initiated at a reaction temperature of 30°C. The reaction was carried out for two hours and then the reaction solution was analyzed. As a result, production of oxidized γ-glutamylcysteine was verified. The yield after a two-hour reaction based on the starting L-cystine was 12 mol% and the yield after a 23-hour reaction was 95 mol%. In this case, the yield of reduced γ-glutamylcysteine after the 23-hour reaction based on the starting L-cystine was 1 mol% or less.

### <Example 4>

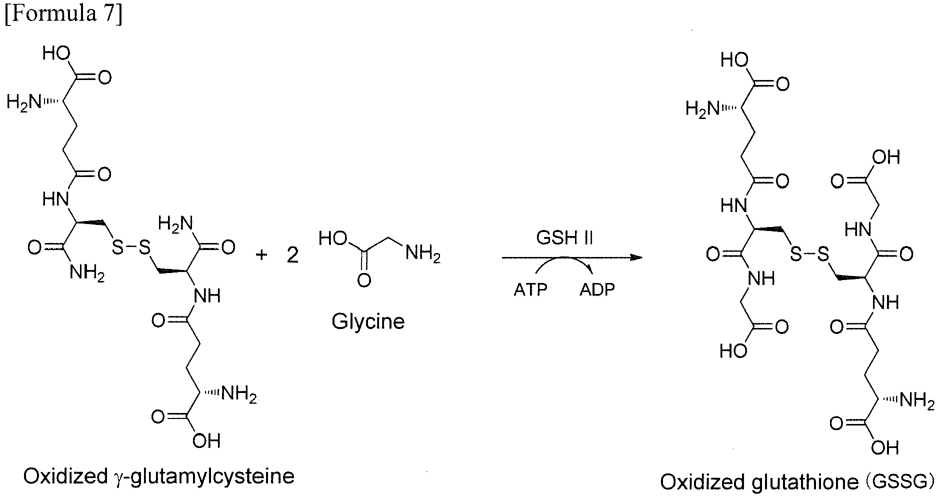

After the 23-hour reaction in Example 3, to the reaction solution, glycine (0.0426 g (0.57 mmol)), magnesium sulfate hepta hydrate (0.2064 g), ATP (0.2392 g (0.4 mmol)) and a glutathione synthetase (GSH II) solution (2 g) prepared in Experiment 2 were added and a reaction was initiated. In this case, the pH of the mixture was controlled to be 7.5 with a 15 wt% aqueous sodium hydroxide solution (0.2 g)). The reaction was carried out for one hour and then the reaction solution was analyzed. As a result, production of oxidized glutathione was verified. The yield after a one-hour reaction based on the starting L-cystine was 6 mol% and the yield after a 5-hour reaction was 70 mol%. In this case, the yield of reduced glutathione after the 5 hour reaction based on the starting L-cystine was 1 mol% or less.

### <Example 5>

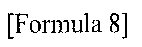

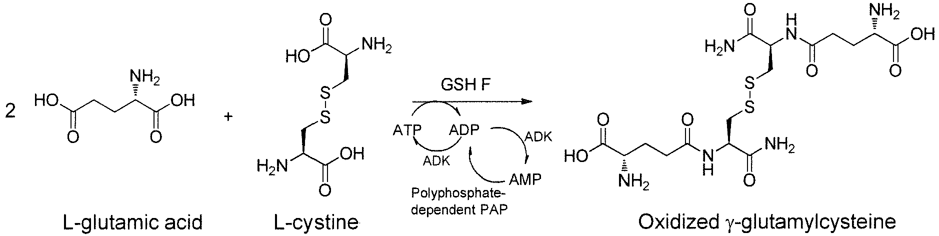

Monosodium L-glutamate monohydrate (0.3662 g (2.17 mmol)), L-cystine dihydrochloride (0.3121 g (1.00 mmol)), magnesium sulfate hepta hydrate (0.7019 g), ATP (0.058 g (0.11 mmol)), sodium metaphosphate (0.8 g) and distilled water (12 g) were mixed. The pH of the mixture was controlled to be 7.5 with a 15 wt% aqueous sodium hydroxide solution (0.8 g). To the mixture, the bifunctional glutathione synthetase (GSH F) solution (2 g) prepared in Experiment 3 and the PAP solution (2 g) prepared in Experiment 4 were added and a reaction was initiated at a reaction temperature of 30°C. The reaction was carried out for one hour and then the reaction solution was analyzed. As a result, production of oxidized γ-glutamylcysteine was verified. The yield after a one-hour reaction based on the starting L-cystine was 9 mol% and the yield after a 19-hour reaction was 61 mol%. In this case, the yield of reduced γ-glutamylcysteine after the 19-hour reaction based on the starting L-cystine was 1 mol% or less.

Since ADK derived from *Escherichia coli* used as a host cell was contained in the GSH F solution prepared in Experiment 3 and the PAP solution prepared in Experiment 4, it was not necessary to separately add ADK.

### Sequence Listing Free Text

SEQ ID NO: 2: primer
SEQ ID NO: 3: primer
SEQ ID NO: 5: primer
SEQ ID NO: 6: primer

All publications including Patents and Patent Applications cited in the specification are incorporated in the specification by reference in their entirety.

## Claims

1. A method for producing oxidized γ-glutamylcysteine, comprising step A' of reacting L-cystine and L-glutamic acid to produce oxidized γ-glutamylcysteine.

2. The method according to Claim 1, wherein the step A' is step A of reacting L-cystine and L-glutamic acid in the presence of at least one enzyme selected from the group consisting of γ-glutamylcysteine synthetase and bifunctional glutathione synthetase, and adenosine triphosphate (ATP), to produce oxidized γ-glutamylcysteine.

3. The method according to Claim 2, wherein the step A is carried out in conjugation with an ATP regeneration reaction for regenerating adenosine diphosphate (ADP) into ATP.

4. The method according to Claim 2 or 3, wherein the γ-glutamylcysteine synthetase is derived from *Escherichia coli.*

5. The method according to Claim 2 or 3, wherein the bifunctional glutathione synthetase is derived from *Streptococcus agalactiae.*

6. A method for producing oxidized glutathione, comprising step B' of reacting oxidized γ-glutamylcysteine and glycine to produce oxidized glutathione.

7. The method according to Claim 6, wherein the step B' is step B of reacting oxidized γ-glutamylcysteine and glycine in the presence of glutathione synthetase and adenosine triphosphate (ATP) to produce oxidized glutathione.

8. The method according to Claim 7, wherein the step B is carried out in conjugation with an ATP regeneration reaction for regenerating adenosine diphosphate (ADP) into ATP.

9. The method according to Claim 7 or 8, wherein the glutathione synthetase is derived from *Escherichia coli.*

10. The method according to any one of Claims 6 to 9, further comprising step A' of reacting L-cystine and L-glutamic acid to produce oxidized γ-glutamylcysteine, wherein the oxidized γ-glutamylcysteine used in the step B' is produced by the step A'.

11. The method according to Claim 10, wherein the step A' is step A of reacting L-cystine and L-glutamic acid in the presence of at least one enzyme selected from the group consisting of γ-glutamylcysteine synthetase and bifunctional glutathione synthetase, and adenosine triphosphate (ATP), to produce oxidized γ-glutamylcysteine.

12. The method according to Claim 11, wherein the step A is carried out in conjugation with an ATP regeneration reaction for regenerating adenosine diphosphate (ADP) into ATP.

13. The method according to Claim 11 or 12, wherein the γ-glutamylcysteine synthetase is derived from *Escherichia coli.*

14. The method according to Claim 11 or 12, wherein the bifunctional glutathione synthetase is derived from *Streptococcus agalactiae.*
